# EUROPEAN PATENT APPLICATION

(11) **EP 3 988 653 A2**
(43) Date of publication of application: **27.04.2022**
(21) Application number: 21190194.7
(22) Date of filing: 23.03.2016
(51) Int. Cl.: C12N 9/10, C12N 9/02, C12P 19/56, A23L 27/30, A23L 2/60

(54) **UDP-GLYCOSYLTRANSFERASES FROM SOLANUM LYCOPERSICUM**

(30) Priority: 23.03.2015 US 201562136759 P
(62) Divisional of application: 16711641.7
(71) Applicant: DSM IP Assets B.V., 6411 TE Heerlen (NL)
(72) Inventor: BOSCH, Hendrik Jan, 6100 AA Echt (NL); BEEKWILDER, Martinus Julius, 6100 AA Echt (NL); BOER, Viktor Marius, 6100 AA Echt (NL)
(74) Representative: DSM Intellectual Property

(57) **Abstract**

The present invention relates to polypeptides having UDP-Glycosyltransferase activity derived from tomato (Solanum lycopersicum) and having the amino acid sequence set out in any of SEQ ID NO: 1 to 4 or an amino acid sequence having at least about 30% sequence identity thereto. The application also relates to recombinant hosts comprising a recombinant nucleic acid sequence encoding said polypeptides and uses thereof to prepare glycosylated diterpenes, like steviol glycoside. The host cells might comprise further enzymes of the steviol glycoside biosynthesis pathway.

## Description

### Field

The present invention relates to a recombinant host comprising a recombinant nucleic acid sequence encoding a UDP-glycosyltransferase (UGT) polypeptide. The invention also relates to a process for the preparation of a glycosylated diterpene using such a recombinant host and to a fermentation broth which may be the result of such a process. The invention further relates to a glycosylated diterpene obtained by such a process or obtainable from such a fermentation broth and to a composition comprising two or more such glycosylated diterpenes. In addition the invention relates to a foodstuff, feed or beverage which comprises such a glycosylated diterpene or a such composition. The invention also relates to a method for converting a first glycosylated diterpene into a second glycosylated diterpene using the above-mentioned recombinant host.

### Background

The leaves of the perennial herb, *Stevia rebaudiana* Bert., accumulate quantities of intensely sweet compounds known as steviol glycosides. Whilst the biological function of these compounds is unclear, they have commercial significance as alternative high potency sweeteners.

These sweet steviol glycosides have functional and sensory properties that appear to be superior to those of many high potency sweeteners. In addition, studies suggest that stevioside can reduce blood glucose levels in Type II diabetics and can reduce blood pressure in mildly hypertensive patients.

Steviol glycosides accumulate in Stevia leaves where they may comprise from 10 to 20% of the leaf dry weight. Stevioside and rebaudioside A are both heat and pH stable and suitable for use in carbonated beverages and many other foods. Stevioside is between 110 and 270 times sweeter than sucrose, rebaudioside A between 150 and 320 times sweeter than sucrose. In addition, rebaudioside D is also a high-potency diterpene glycoside sweetener which accumulates in Stevia leaves. It may be about 200 times sweeter than sucrose. Rebaudioside M is a further high-potency diterpene glycoside sweetener. It is present in trace amounts in certain stevia variety leaves, but has been suggested to have a superior taste profile.

Steviol glycosides have traditionally been extracted from the *Stevia* plant. In *Stevia,* (-)-kaurenoic acid, an intermediate in gibberellic acid (GA) biosynthesis, is converted into the tetracyclic diterpene steviol, which then proceeds through a multi-step glycosylation pathway to form the various steviol glycosides. However, yields may be variable and affected by agriculture and environmental conditions. Also, Stevia cultivation requires substantial land area, a long time prior to harvest, intensive labour and additional costs for the extraction and purification of the glycosides.

More recently, interest has grown in producing steviol glycosides using fermentative processes. WO2013/110673 and WO2015/007748 describe microorganisms that may be used to produce at least the steviol glycosides rebaudioside A and rebaudioside D.

Further improvement of such microorganisms is desirable in order that higher amounts of steviol glycosides may be produced and/or additional or new steviol glycosides and/or higher amounts of specific steviol glycosides and/or mixtures of steviol glycosides having desired ratios of different steviol glycosides.

### Summary

In *Stevia rebaudiana,* steviol is synthesized from GGPP, which is formed by the deoxyxylulose 5- phosphate pathway. The activity of two diterpene cyclases (-)-copalyl diphosphate synthase (CPS) and (-)-kaurene synthase (KS) results in the formation of (-)-Kaurene which is then oxidized in a three step reaction by (-)-kaurene oxidase (KO) to form (-)-kaurenoic acid.

In *Stevia rebaudiana* leaves, (-)-kaurenoic acid is then hydroxylated, by ent-kaurenoic acid 13-hydroxylase (KAH) to form steviol. Steviol is then glycosylated by a series of UDP-glycosyltransferases (UGTs) leading to the formation of a number of steviol glycosides. Specifically, these molecules can be viewed as a steviol molecule, with its carboxyl hydrogen atom replaced by a glucose molecule to form an ester, and an hydroxyl hydrogen with combinations of glucose and rhamnose to form an acetal.

These pathways may be reconstructed in recombinant hosts, for example yeasts such as yeasts of the genera *Saccharomyces* and *Yarrowia.*

The invention relates to the identification of polypeptides having UDP-glycosyltransferase (UGT), typically having improved properties in comparison to those that are currently known. These polypeptides may be used to generate recombinant hosts that produce higher amounts of steviol glycosides and/or additional or new steviol glycosides and/or higher amounts of specific steviol glycosides and/or mixtures of steviol glycosides having desired ratios of different steviol glycosides.

Thus, the invention also relates to a recombinant host capable of producing a glycosylated diterpene, i.e. a diterpene glycoside such as a steviol glycoside, for example steviolmonoside, steviolbioside, stevioside, rebaudioside A, rebaudioside B, rebaudioside C, rebaudioside D, rebaudioside E, rebaudioside F, rebaudioside M, rubusoside, dulcoside A, steviol-13-monoside, steviol-19-monoside or 13-[(β-D-Glucopyranosyl)oxy)kaur-16-en-18-oic acid 2-O-β-D-glucopyranosyl-p-D-glucopyranosyl ester steviol-19-diside.

Accordingly, the invention relates to a recombinant host comprising a recombinant nucleic acid sequence, typically having UDP-glycosyltransferase (UGT) activity such as UGT2 activity, encoding a polypeptide having:
a. the amino acid sequence set forth in SEQ ID NO: 1 or an amino acid sequence having at least about 30% sequence identity thereto;
b. the amino acid sequence set forth in SEQ ID NO: 2 or an amino acid sequence having at least about 30% sequence identity thereto;
c. the amino acid sequence set forth in SEQ ID NO: 3 or an amino acid sequence having at least about 30% sequence identity thereto; or
d. the amino acid sequence set forth in SEQ ID NO: 4 or an amino acid sequence having at least about 30% sequence identity thereto.

The invention also relates to:
- a process for the preparation of a glycosylated diterpene which comprises fermenting a recombinant host of the invention in a suitable fermentation medium, and optionally recovering the glycosylated diterpene;
- a fermentation broth comprising a glycosylated diterpene obtainable by the process of the invention;
- a glycosylated diterpene obtained by such a process or obtainable from such a fermentation broth;
- a composition comprising two or more such diterpenes;
- a foodstuff, feed or beverage which comprises such a glycosylated diterpene; and
- a method for converting a first glycosylated diterpene into a second glycosylated diterpene, which method comprises:
   - contacting said first glycosylated diterpene with a recombinant host of the invention, a cell free extract derived from such a recombinant host or an enzyme preparation derived from either thereof;
   - thereby to convert the first glycosylated diterpene into the second glycosylated diterpene.

### Brief description of the drawings

Figure 1 sets out Western blot detection of His-tagged UGTs
Figure 2 sets out Western blot of UGT2_1a and RT18. Lanes 1,2,3,4: 0.5, 1.0, 1.9, 3.8 µg of UGT2_1a crude enzyme extract. Lane 5 and 6: 31.9 and 63.8 µg RT18 crude enzyme extract.
Figure 3 sets out the effect of the expression of RT18 on the production of RebM
Figure 4 sets out the effect of the expression of RT18 on the production of RebD
Figure 5 sets out a schematic diagram of the potential pathways leading to biosynthesis of steviol glycosides.
Figure 6 sets out a schematic diagram of the potential pathways leading to biosynthesis of steviol glycosides. The compound shown with an asterisk is 13-[(β-D-Glucopyranosyl)oxy)kaur-16-en-18-oic acid 2-O-β-D-glucopyranosyl-β-D-glucopyranosyl ester.

### Description of the sequence listing

A description of the sequences is set out in Table 10. Sequences described herein may be defined with reference to the sequence listing or with reference to the database accession numbers also set out herein, for example in Table 10.

### Detailed description

Throughout the present specification and the accompanying claims, the words "comprise", "include" and "having" and variations such as "comprises", "comprising", "includes" and "including" are to be interpreted inclusively. That is, these words are intended to convey the possible inclusion of other elements or integers not specifically recited, where the context allows.

The articles "a" and "an" are used herein to refer to one or to more than one (i.e. to one or at least one) of the grammatical object of the article. By way of example, "an element" may mean one element or more than one element.

Herein, "rebaudioside" may be shortened to "reb". That is to say, rebaudioside A and reb A, for example, are intended to indicate the same molecule.

The term "recombinant" when used in reference to a cell, nucleic acid, protein or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell or express native genes that are otherwise abnormally expressed, under expressed or not expressed at all. The term "recombinant" is synonymous with "genetically modified".

The invention concerns polypeptides identified as having UDP-glycosyltransferase (UGT) activity which can be used in recombinant hosts, typically for the production of diterpene glycosides, such as steviol glycosides.

For the purposes of this invention, a polypeptide having UGT activity is one which has glycosyltransferase activity (EC 2.4), i.e. that can act as a catalyst for the transfer of a monosaccharide unit from an activated nucleotide sugar (also known as the "glycosyl donor") to a glycosyl acceptor molecule, usually an alcohol. The glycosyl donor for a UGT is typically the nucleotide sugar uridine diphosphate glucose (uracil-diphosphate glucose, UDP-glucose). A polypeptide suitable for use in a host of the invention typically has UGT activity and a polynucleotide sequence as described herein typically encodes such a polypeptide. Typically, the polypeptides for use in a host of the invention are polypeptides having UGT2-type activity.

The invention thus provides a recombinant host comprising a recombinant nucleic acid sequence encoding a polypeptide comprising:
a. the amino acid sequence set forth in SEQ ID NO: 1 or an amino acid sequence having at least about 30% sequence identity thereto;
b. the amino acid sequence set forth in SEQ ID NO: 2 or an amino acid sequence having at least about 30% sequence identity thereto;
c. the amino acid sequence set forth in SEQ ID NO: 3 or an amino acid sequence having at least about 30% sequence identity thereto; or
d. the amino acid sequence set forth in SEQ ID NO: 4 or an amino acid sequence having at least about 30% sequence identity thereto.

The polypeptide encoded by the recombinant nucleic acid sequence typically has UGT activity, such as UGT2 activity. A recombinant host of the invention is typically capable of producing a glycosylated diterpene, for example a steviol glycoside.

A polypeptide encoded by a recombinant nucleic acid present in a recombinant host of the invention may comprise an amino acid sequence having at least about 35%, at least about 40%, at least about 50%, at least about 60%, at least about 65%, at least about 70%, at least about 75%, at least about 80%, at least about 85%, at least about, 86%, at least about 87%, at least about 88%, at least about 89%, at least about 90%, at least about 91%, at least about 92%, at least about 93%, at least about 94%, at least about 95%, at least about 96%, at least about 97%, at least about 98% or at least about 99% sequence identity to any one of SEQ ID NOs: 1, 2, 3 or 4.

Thus, the invention relates to:
- a recombinant host comprising a recombinant nucleic acid sequence encoding a polypeptide, typically having UGT activity, which comprises an amino acid sequence having at least about 35%, at least about 40%, at least about 50%, at least about 60%, at least about 65%, at least about 70%, at least about 75%, at least about 80%, at least about 85%, at least about, 86%, at least about 87%, at least about 88%, at least about 89%, at least about 90%, at least about 91%, at least about 92%, at least about 93%, at least about 94%, at least about 95%, at least about 96%, at least about 97%, at least about 98% or at least about 99% sequence identity to SEQ ID NO: 1;
- a recombinant host comprising a recombinant nucleic acid sequence encoding a polypeptide, typically having UGT activity, which comprises an amino acid sequence having at least about 35%, at least about 40%, at least about 50%, at least about 60%, at least about 65%, at least about 70%, at least about 75%, at least about 80%, at least about 85%, at least about, 86%, at least about 87%, at least about 88%, at least about 89%, at least about 90%, at least about 91%, at least about 92%, at least about 93%, at least about 94%, at least about 95%, at least about 96%, at least about 97%, at least about 98% or at least about 99% sequence identity to SEQ ID NO: 2;
- a recombinant host comprising a recombinant nucleic acid sequence encoding a polypeptide, typically having UGT activity, which comprises an amino acid sequence having at least about 35%, at least about 40%, at least about 50%, at least about 60%, at least about 65%, at least about 70%, at least about 75%, at least about 80%, at least about 85%, at least about, 86%, at least about 87%, at least about 88%, at least about 89%, at least about 90%, at least about 91%, at least about 92%, at least about 93%, at least about 94%, at least about 95%, at least about 96%, at least about 97%, at least about 98% or at least about 99% sequence identity to SEQ ID NO: 3;
- a recombinant host comprising a recombinant nucleic acid sequence encoding a polypeptide, typically having UGT activity, which comprises an amino acid sequence having at least about 35%, at least about 40%, at least about 50%, at least about 60%, at least about 65%, at least about 70%, at least about 75%, at least about 80%, at least about 85%, at least about, 86%, at least about 87%, at least about 88%, at least about 89%, at least about 90%, at least about 91%, at least about 92%, at least about 93%, at least about 94%, at least about 95%, at least about 96%, at least about 97%, at least about 98% or at least about 99% sequence identity to SEQ ID NO: 4.

As used herein, the term "polypeptide" refers to a molecule comprising amino acid residues linked by peptide bonds and containing more than five amino acid residues. The amino acids are identified by either the single-letter or three-letter designations. The term "protein" as used herein is synonymous with the term "polypeptide" and may also refer to two or more polypeptides. Thus, the terms "protein", "peptide" and "polypeptide" can be used interchangeably. Polypeptides may optionally be modified (e.g., glycosylated, phosphorylated, acylated, farnesylated, prenylated, sulfonated, and the like) to add functionality. Polypeptides exhibiting activity may be referred to as enzymes. It will be understood that, as a result of the degeneracy of the genetic code, a multitude of nucleotide sequences encoding a given polypeptide may be produced.

The term "nucleic acid sequence" (or ""polynucleotide") as used in the present invention refers to a nucleotide polymer including at least 5 nucleotide units. A nucleic acid refers to a ribonucleotide polymer (RNA), deoxynucleotide polymer (DNA) or a modified form of either type of nucleic acid or synthetic form thereof or mixed polymers of any of the above. Nucleic acids may include either or both naturally-occurring and modified nucleic acids linked together by naturally-occurring and/or non-naturally occurring nucleic acid linkages. The nucleic acid molecules may be modified chemically or biochemically or may contain non-natural or derivatized nucleic acid bases, as will be readily appreciated by those of skill in the art. Such modifications include, for example, labels, methylation, substitution of one or more of the naturally occurring nucleic acids with an analog, internucleotide modifications such as uncharged linkages (*e.g.,* methyl phosphonates, phosphotriesters, phosphoramidates, carbamates, etc.), charged linkages (*e.g.,* phosphorothioates, phosphorodithioates, etc.), pendent moieties (*e.g.,* polypeptides), intercalators (*e.g.,* acridine, psoralen, etc.), chelators, alkylators, and modified linkages (*e.g.,* alpha anomeric nucleic acids, etc.) The term nucleic acid is also intended to include any topological conformation, including single-stranded (sense strand and antisense strand), double-stranded, partially duplexed, triplex, hairpinned, circular and padlocked conformations. Also included are synthetic molecules that mimic nucleic acids in their ability to bind to a designated sequence via hydrogen bonding and other chemical interactions. Such molecules are known in the art and include, for example, those in which peptide linkages substitute for phosphate linkages in the backbone of the molecule. A reference to a nucleic acid sequence encompasses its complement unless otherwise specified. Thus, a reference to a nucleic acid molecule having a particular sequence should be understood to encompass its complementary strand, with its complementary sequence. The complementary strand is also useful, e.g., for antisense therapy, hybridization probes and PCR primers. The term "nucleic acid", "polynucleotide" and "polynucleotide sequence" can be used interchangeably herein. A polypeptide encoded by a recombinant nucleic acid for use in a recombinant host of the invention may comprise a signal peptide and/or a propeptide sequence. In the event that a polypeptide comprises a signal peptide and/or a propeptide, sequence identity may be calculated over the mature polypeptide sequence.

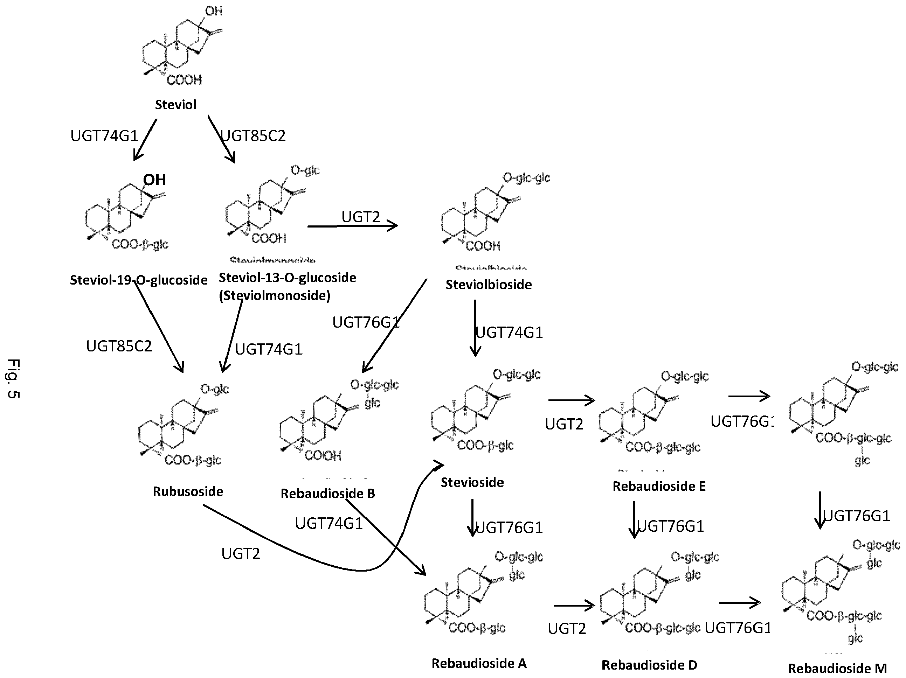

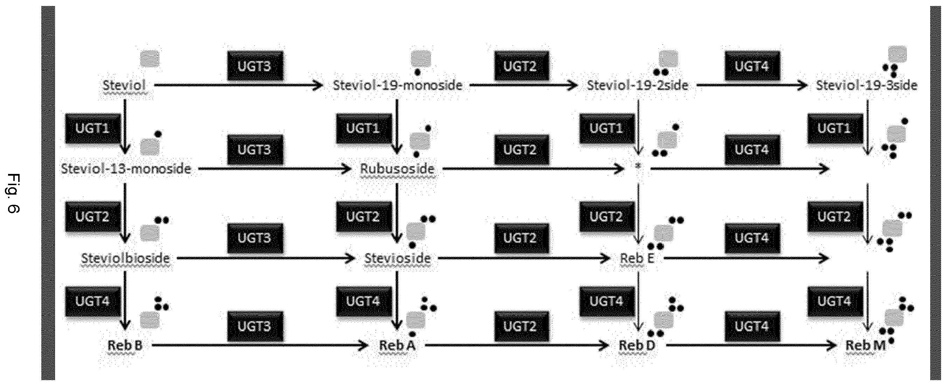

The polypeptide typically has UGT activity and more preferably has UGT2 activity. Figures 5 and 6 illustrate a non-exhaustive list of reactions that may be catalyzed by a polypeptide having UGT2 activity.

A polypeptide having UGT2 activity is one which may function as a uridine 5'-diphospho glucosyl: steviol- 13-O-glucoside transferase (also referred to as a steviol-13- monoglucoside 1,2-glucosylase), transferring a glucose moiety to the C-2' of the 13- O-glucose of the acceptor molecule, steviol- 13-O-glucoside. Typically, a suitable UGT2 polypeptide may also function as a uridine 5'-diphospho glucosyl: rubusoside transferase transferring a glucose moiety to the C-2' of the 13-0-glucose of the acceptor molecule, rubusoside. That is to say be capable of converting steviol-13-monoside to steviolbioside and/or capable of converting rubusoside to stevioside.

A polypeptide having UGT2 activity may also or alternatively catalyze reactions that utilize steviol glycoside substrates other than steviol- 13-O-glucoside and rubusoside, e.g., a functional UGT2 polypeptide may utilize stevioside as a substrate, transferring a glucose moiety to the C-2' of the 19-O-glucose residue to produce rebaudioside E. A functional UGT2 polypeptide may also or alternatively utilize rebaudioside A as a substrate, transferring a glucose moiety to the C-2' of the 19-O-glucose residue to produce rebaudioside D.

A polypeptide having UGT2 activity may also catalyze reactions that utilize steviol-19-glucoside or rubusoside as a substrate, e.g., a functional UGT2 polypeptide may utilize steviol-19-glucoside or rubusoside as a substrate, transferring a glucose moiety to the 19 position to produce steviol-19-2side or 13-[(β-D-Glucopyranosyl)oxy)kaur-16-en-18-oic acid 2-O-β-D-glucopyranosyl-β-D-glucopyranosyl ester respectively.

However, a functional UGT2 polypeptide typically does not transfer a glucose moiety to steviol compounds having a 1,3-bound glucose at the C- 13 position, i.e., transfer of a glucose moiety to steviol 1,3-bioside and 1,3-stevioside typically does not occur.

A polypeptide having UGT2 activity may also or alternatively transfer sugar moieties from donors other than uridine diphosphate glucose. For example, a polypeptide having UGT2 activity act as a uridine 5'-diphospho D-xylosyl: steviol- 13 -O-glucoside transferase, transferring a xylose moiety to the C-2' of the 13-O-glucose of the acceptor molecule, steviol- 13 -O-glucoside. As another example, a polypeptide having UGT2 activity may act as a uridine 5'-diphospho L-rhamnosyl: steviol- 13-O-glucoside transferase, transferring a rhamnose moiety to the C-2' of the 13-O-glucose of the acceptor molecule, steviol.

One or more of the above-described activities may be used to define a polypeptide having UGT2 activity encoded by a recombinant nucleic acid sequence for use in a recombinant host of the invention. Such a polypeptide may have improved UGT2 activity in respect of one or more of the above-described activities in comparison with the UGT2_1a polypeptide (SEQ ID NO: 6).

A polynucleotide encoding a polypeptide for use in a recombinant host of the invention may be used to steer production of steviol glycosides in a recombinant cell to a desired steviol glycoside, such as rebaudioside A, rebaudioside D or rebaudioside M. For example, a UGT2 polypeptide which preferentially catalyzes conversion of steviol-13-monoside to steviolbioside and/or conversion of rubusoside to stevioside may help to steer production towards rebaudioside A, whereas a UGT2 polypeptide which preferentially catalyzes conversion of stevioside to reb E or rubusoside to a compound with an additional sugar at the 19 position may help to steer production towards rebaudioside M. That is to say preference for addition of a sugar moiety at the 13 position may help steer production towards rebaudioside A, whereas preference for addition of a sugar moiety at the 19 position may help steer production towards rebaudioside M.

A recombinant nucleic acid sequence for use in a recombinant host of the invention may be provided in the form of a nucleic acid construct. The term "nucleic acid construct" refers to as a nucleic acid molecule, either single-or double-stranded, which is isolated from a naturally occurring gene or which has been modified to contain segments of nucleic acid which are combined and juxtaposed in a manner which would not otherwise exist in nature. The term nucleic acid construct is synonymous with the term "expression cassette" when the nucleic acid construct contains all the control sequences required for expression of a coding sequence, wherein said control sequences are operably linked to said coding sequence.

A recombinant nucleic acid sequence for use in a recombinant host of the invention may be provided in the form of an expression vector, wherein the polynucleotide sequence is operably linked to at least one control sequence for the expression of the polynucleotide sequence in a recombinant host cell.

The term "operably linked" as used herein refers to two or more nucleic acid sequence elements that are physically linked and are in a functional relationship with each other. For instance, a promoter is operably linked to a coding sequence if the promoter is able to initiate or regulate the transcription or expression of a coding sequence, in which case the coding sequence should be understood as being "under the control of" the promoter. Generally, when two nucleic acid sequences are operably linked, they will be in the same orientation and usually also in the same reading frame. They usually will be essentially contiguous, although this may not be required.

An expression vector comprises a polynucleotide coding for a polypeptide as described herein, operably linked to the appropriate control sequences (such as a promoter, and transcriptional and translational stop signals) for expression and/or translation *in vitro,* or in the host cell of the polynucleotide.

The expression vector may be any vector (e.g., a plasmid or virus), which can be conveniently subjected to recombinant DNA procedures and can bring about the expression of the polynucleotide. The choice of the vector will typically depend on the compatibility of the vector with the cell into which the vector is to be introduced. The vectors may be linear or closed circular plasmids. The vector may be an autonomously replicating vector, i.e., a vector, which exists as an extra-chromosomal entity, the replication of which is independent of chromosomal replication, e.g., a plasmid, an extra-chromosomal element, a mini-chromosome, or an artificial chromosome.

Alternatively, the vector may be one which, when introduced into the host cell, is integrated into the genome and replicated together with the chromosome(s) into which it has been integrated. The integrative cloning vector may integrate at random or at a predetermined target locus in the chromosomes of the host cell. A vector may comprise one or more selectable markers, which permit easy selection of transformed cells.

Standard genetic techniques, such as overexpression of enzymes in the host cells, as well as for additional genetic modification of host cells, are known methods in the art, such as described in Sambrook and Russel (2001) "Molecular Cloning: A Laboratory Manual (3rd edition), Cold Spring Harbor Laboratory, Cold Spring Harbor Laboratory Press*,* or F. Ausubel et al, eds., "Current protocols in molecular biology", Green Publishing and Wiley Interscience, New York (1987). Methods for transformation and genetic modification of fungal host cells are known from e.g. EP-A-0 635 574, WO 98/46772, WO 99/60102 and WO 00/37671.

A recombinant host of the invention may comprise any polypeptide as described herein. Typically, a recombinant host of the invention is capable of producing a glycosylated diterpene, such as a steviol glycoside. For example, a recombinant host of the invention may be capable of producing one or more of, for example, steviol-13-monoside, steviol-19-monoside, 13-[(β-D-Glucopyranosyl)oxy)kaur-16-en-18-oic acid 2-O-β-D-glucopyranosyl-β-D-glucopyranosyl ester, rubusoside, stevioside, steviol-19-diside, steviolbioside, reb A, reb E, reb D or reb M.

Thus, a recombinant host of the invention will typically comprise polynucleotides encoding polypeptides having UGT1, UGT2, UGT2 and UGT4 activity and polypeptides which provide for the production of steviol in the host (which may then be converted to one or more steviol glycosides).

One polynucleotide may encode more than one of such polypeptides. One polynucleotide may encode a polypeptide having more than one of the activities UGT1, UGT2, UGT3 or UGT4 or the activity of a polypeptide providing for production of steviol in the host. Accordingly, a recombinant host according to the invention may comprise one or more recombinant nucleotide sequence(s) encoding one of more of:
a polypeptide having ent-copalyl pyrophosphate synthase activity;
a polypeptide having ent-Kaurene synthase activity;
a polypeptide having ent-Kaurene oxidase activity; and
a polypeptide having kaurenoic acid 13-hydroxylase activity.

A recombinant host may comprise one or more recombinant polynucleotide sequences encoding all four such polypeptides.

For the purposes of this invention, a polypeptide having *ent*-copalyl pyrophosphate synthase (EC 5.5.1.13) is capable of catalyzing the chemical reaction:

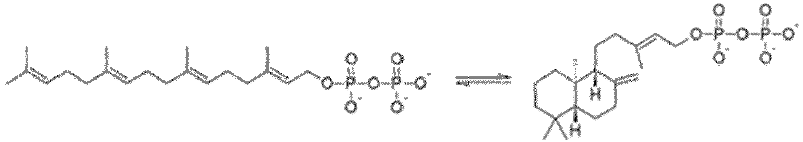

This enzyme has one substrate, geranylgeranyl pyrophosphate, and one product, *ent-*copalyl pyrophosphate. This enzyme participates in gibberellin biosynthesis. This enzyme belongs to the family of isomerase, specifically the class of intramolecular lyases. The systematic name of this enzyme class is *ent*-copalyl-diphosphate lyase (decyclizing). Other names in common use include having *ent*-copalyl pyrophosphate synthase, *ent*-kaurene synthase A, and *ent*-kaurene synthetase A.

Suitable nucleic acid sequences encoding an ent-copalyl pyrophosphate synthase may for instance comprise a sequence as set out in SEQ ID. NO: 1, 3, 5, 7, 17, 19, 59, 61, 141, 142, 151, 152, 153, 154, 159, 160, 182 or 184 of WO2015/007748.

For the purposes of this invention, a polypeptide having ent-kaurene synthase activity (EC 4.2.3.19) is a polypeptide that is capable of catalyzing the chemical reaction:
ent-copalyl diphosphate ⇄ent-kaurene + diphosphate

Hence, this enzyme has one substrate, ent-copalyl diphosphate, and two products, ent-kaurene and diphosphate.

This enzyme belongs to the family of lyases, specifically those carbon-oxygen lyases acting on phosphates. The systematic name of this enzyme class is ent-copalyl-diphosphate diphosphate-lyase (cyclizing, ent-kaurene-forming). Other names in common use include ent-kaurene synthase B, ent-kaurene synthetase B, ent-copalyl-diphosphate diphosphate-lyase, and (cyclizing). This enzyme participates in diterpenoid biosynthesis.

Suitable nucleic acid sequences encoding an ent-Kaurene synthase may for instance comprise a sequence as set out in SEQ ID. NO: 9, 11, 13, 15, 17, 19, 63, 65, 143, 144, 155, 156, 157, 158, 159, 160, 183 or 184 of WO2015/007748.

*ent*-copalyl diphosphate synthases may also have a distinct *ent*-kaurene synthase activity associated with the same protein molecule. The reaction catalyzed by *ent*-kaurene synthase is the next step in the biosynthetic pathway to gibberellins. The two types of enzymic activity are distinct, and site-directed mutagenesis to suppress the *ent*-kaurene synthase activity of the protein leads to build up of *ent*-copalyl pyrophosphate.

Accordingly, a single nucleotide sequence used in a recombinant host of the invention may encode a polypeptide having *ent*-copalyl pyrophosphate synthase activity and *ent*-kaurene synthase activity. Alternatively, the two activities may be encoded two distinct, separate nucleotide sequences.

For the purposes of this invention, a polypeptide having ent-kaurene oxidase activity (EC 1.14.13.78) is a polypeptide which is capable of catalysing three successive oxidations of the 4-methyl group of ent-kaurene to give kaurenoic acid. Such activity typically requires the presence of a cytochrome P450.

Suitable nucleic acid sequences encoding an ent-Kaurene oxidase may for instance comprise a sequence as set out in SEQ ID. NO: 21, 23, 25, 67, 85, 145, 161, 162, 163, 180 or 186 of WO2015/007748.

For the purposes of the invention, a polypeptide having kaurenoic acid 13-hydroxylase activity (EC 1.14.13) is one which is capable of catalyzing the formation of steviol (ent-kaur-16-en-13-ol-19-oic acid) using NADPH and O₂. Such activity may also be referred to as ent-ka 13-hydroxylase activity.

Suitable nucleic acid sequences encoding a kaurenoic acid 13-hydroxylase may for instance comprise a sequence as set out in SEQ ID. NO: 27, 29, 31, 33, 69, 89, 91, 93, 95, 97, 146, 164, 165, 166, 167 or 185 of WO2015/007748.

A recombinant host of the invention may comprise a recombinant nucleic acid sequence encoding a polypeptide having NADPH-cytochrome p450 reductase activity. That is to say, a recombinant host of the invention may be capable of expressing a nucleotide sequence encoding a polypeptide having NADPH-cytochrome p450 reductase activity. For the purposes of the invention, a polypeptide having NADPH-Cytochrome P450 reductase activity (EC 1.6.2.4; also known as NADPH:ferrihemoprotein oxidoreductase, NADPH:hemoprotein oxidoreductase, NADPH:P450 oxidoreductase, P450 reductase, POR, CPR, CYPOR) is typically one which is a membrane-bound enzyme allowing electron transfer to cytochrome P450 in the microsome of the eukaryotic cell from a FAD- and FMN-containing enzyme NADPH:cytochrome P450 reductase (POR; EC 1.6.2.4).

A recombinant host of the invention may comprise one or more recombinant nucleic acid sequences encoding one or more UGT polypeptides, in addition to RT7, RT11, RT15 or RT18 or related sequences as described herein. Such additional UGTs may be selected so as to produce a desired diterpene glycoside, such as a steviol glycoside. Schematic diagrams of steviol glycoside formation are set out in Humphrey et al., Plant Molecular Biology (2006) 61: 47-62 and Mohamed et al., J. Plant Physiology 168 (2011) 1136-1141. In addition, Figures 5 and 6 sets out a schematic diagram of steviol glycoside formation.

A recombinant host of the invention may thus comprise one or more recombinant nucleic acid sequences encoding one or more of:
(i) a polypeptide having UGT74G1 activity (UGT3 activity);
(ii) a polypeptide having UGT85C2 activity (UGT1 activity); and
(iii) a polypeptide having UGT76G1 activity (UGT4 activity).

Figures 5 and 6 set out schematic diagram of the potential pathways leading to biosynthesis of steviol glycosides.

A recombinant host of the invention will typically comprise at least one recombinant nucleic acid encoding a polypeptide having UGT1 activity, at least one recombinant nucleic acid encoding a polypeptide having UGT2 activity, at least one recombinant nucleic acid encoding a polypeptide having UGT3 activity and at least one recombinant nucleic acid encoding a polypeptide having UGT4 activity. One nucleic acid may encode two or more of such polypeptides.

A recombinant host of the invention typically comprises polynucleotides expressing at least one of each of a UGT1, UGT2, UGT3 and UGT4 polypeptide and a polypeptide having ent-copalyl pyrophosphate synthase activity, a polypeptide having ent-Kaurene synthase activity, a polypeptide having ent-Kaurene oxidase activity and a polypeptide having kaurenoic acid 13-hydroxylase activity. In such a recombinant host, all polynucleotides encoding such polypeptides may be recombinant.

A nucleic acid encoding a polypeptide as described herein may be used to steer production of steviol glycosides in a recombinant cell to a desired steviol glycoside, such as rebaudioside A, rebaudioside D or rebaudioside M. For example, a recombinant nucleic acid which encodes a UGT2 polypeptide which preferentially catalyzes conversion of steviol-13-monoside to steviolbioside and/or conversion of rubusoside to stevioside may help to steer production towards rebaudioside A, whereas a recombinant nucleic acid which encodes a UGT2 polypeptide which preferentially catalyzes conversion of stevioside to reb E or rubusoside to a compound with an additional sugar at the 19 position may help to steer production towards rebaudioside M. That is to say preference for addition of a sugar moiety at the 13 position may help steer production towards rebaudioside A, whereas preference for addition of a sugar moiety at the 19 position may help steer production towards rebaudioside M. A recombinant host of the invention may comprises a nucleotide sequence encoding a polypeptide capable of catalyzing the addition of a C-13-glucose to steviol. That is to say, a recombinant host of the invention may comprise a UGT which is capable of catalyzing a reaction in which steviol is converted to steviolmonoside.

Such a recombinant host of the invention may comprise a nucleotide sequence encoding a polypeptide having the activity shown by UDP-glycosyltransferase (UGT) UGT85C2, whereby the nucleotide sequence upon transformation of the host confers on that host the ability to convert steviol to steviolmonoside.

UGT85C2 activity is transfer of a glucose unit to the 13-OH of steviol. Thus, a suitable UGT85C2 may function as a uridine 5'-diphospho glucosyl: steviol 13-OH transferase, and a uridine 5'-diphospho glucosyl: steviol- 19-O-glucoside 13-OH transferase. A functional UGT85C2 polypeptides may also catalyze glucosyl transferase reactions that utilize steviol glycoside substrates other than steviol and steviol- 19-O-glucoside. Such sequences may be referred to as UGT1 sequences herein.

A recombinant host of the invention may comprises a nucleotide sequence encoding a polypeptide having UGT activity may comprise a nucleotide sequence encoding a polypeptide capable of catalyzing the addition of a C-19-glucose to steviolbioside and/or to rebaudioside B. That is to say, a recombinant host of the invention may comprise a UGT which is capable of catalyzing a reaction in which steviolbioside is converted to stevioside and/or in which rebaudioside B is converted to rebaudioside A. Accordingly, such a recombinant host may be capable of converting steviolbioside to stevioside and/or rebaudioside B is converted to rebaudioside A. Expression of such a nucleotide sequence may confer on the recombinant host the ability to produce at least stevioside and/or rebaudioside A.

A recombinant host of the invention may thus also comprise a nucleotide sequence encoding a polypeptide having the activity shown by UDP-glycosyltransferase (UGT) UGT74G1, whereby the nucleotide sequence upon transformation of the host confers on the cell the ability to convert steviolbioside to stevioside.

Suitable UGT74G1 polypeptides may be capable of transferring a glucose unit to the 13-OH or the 19-COOH, respectively, of steviol. A suitable UGT74G1 polypeptide may function as a uridine 5'-diphospho glucosyl: steviol 19-COOH transferase and a uridine 5'-diphospho glucosyl: steviol- 13-O-glucoside 19-COOH transferase. Functional UGT74G1 polypeptides also may catalyze glycosyl transferase reactions that utilize steviol glycoside substrates other than steviol and steviol- 13-0-glucoside, or that transfer sugar moieties from donors other than uridine diphosphate glucose. Such sequences may be referred to herein as UGT3 sequences.

A recombinant host of the invention may comprise a nucleotide sequence encoding a polypeptide capable of catalyzing glucosylation of the C-3' of the glucose at the C-13 position of stevioside. That is to say, a recombinant host of the invention may comprise a UGT which is capable of catalyzing a reaction in which stevioside is converted to rebaudioside A. Accordingly, such a recombinant host may be capable of converting stevioside to rebaudioside A. Expression of such a nucleotide sequence may confer on the host the ability to produce at least rebaudioside A.

A recombinant host of the invention may thus also comprise a nucleotide sequence encoding a polypeptide having the activity shown by UDP-glycosyltransferase (UGT) UGT76G1, whereby the nucleotide sequence upon transformation of a host confers on that host the ability to convert stevioside to rebaudioside A and/or steviolbioside to rebaudioside B.

A suitable UGT76G1 adds a glucose moiety to the C-3' of the C-13-O-glucose of the acceptor molecule, a steviol 1,2 glycoside. Thus, UGT76G1 functions, for example, as a uridine 5'-diphospho glucosyl: steviol 13-0-1,2 glucoside C-3 ' glucosyl transferase and a uridine 5'-diphospho glucosyl: steviol- 19-O-glucose, 13-0-1,2 bioside C-3'glucosyl transferase. Functional UGT76G1 polypeptides may also catalyze glucosyl transferase reactions that utilize steviol glycoside substrates that contain sugars other than glucose, e.g., steviol rhamnosides and steviol xylosides. Such sequences may be referred to herein as UGT4 sequences. A UGT4 may alternatively or in addition be capable of converting Reb D to Reb M.

A recombinant host of the invention typically comprises nucleotide sequences encoding polypeptides having all four UGT activities described above. A given nucleic acid may encode a polypeptide having one or more of the above activities. For example, a nucleic acid encode for a polypeptide which has two, three or four of the activities set out above. Preferably, a recombinant host of the invention comprises UGT1, UGT2 and UGT3 and UGT4 activity. Suitable UGT1, UGT3 and UGT4 sequences are described in in Table 1 of WO2015/007748.

A recombinant host of the invention may comprise a recombinant nucleic acid sequence encoding an additional polypeptide having UGT2 activity. That is to say, a recombinant host of the invention may comprise a nucleic acid sequence encoding a variant UGT2 of the invention and one or more additional, different, variant of the invention or any another, different, UGT2.

Use of a nucleic acid sequence encoding a RT7, RT11, RT15 or RT18 polypeptide (or related polypeptide as described herein) may be useful in improving reb A production in a recombinant host of the invention.

Use of a nucleic acid sequence encoding a RT7, RT11, RT15 or RT18 polypeptide (or related polypeptide as described herein) may be useful in improving reb M production in a recombinant host of the invention.

In a recombinant host of the invention, the ability of the host to produce geranylgeranyl diphosphate (GGPP) may be upregulated. Upregulated in the context of this invention implies that the recombinant host produces more GGPP than an equivalent non-recombinant host.

Accordingly, a recombinant host of the invention may comprise one or more nucleotide sequence(s) encoding hydroxymethylglutaryl-CoA reductase, farnesyl-pyrophosphate synthetase and geranylgeranyl diphosphate synthase, whereby the nucleotide sequence(s) upon transformation of a host confer(s) on that host the ability to produce elevated levels of GGPP. Thus, a recombinant host according to the invention may comprise one or more recombinant nucleic acid sequence(s) encoding one or more of hydroxymethylglutaryl-CoA reductase, farnesyl-pyrophosphate synthetase and geranylgeranyl diphosphate synthase.

Accordingly, a recombinant host of the invention may comprise nucleic acid sequences encoding one or more of:
a polypeptide having hydroxymethylglutaryl-CoA reductase activity;
a polypeptide having farnesyl-pyrophosphate synthetase activity;
a polypeptide having geranylgeranyl diphosphate synthase activity.

A recombinant host of the invention may be, for example, an multicellular organism or a cell thereof or a unicellular organism. A host of the invention may be a prokaryotic, archaebacterial or eukaryotic host cell.

A prokaryotic host cell may, but is not limited to, a bacterial host cell. An eukaryotic host cell may be, but is not limited to, a yeast, a fungus, an amoeba, an algae, an animal, an insect or a plant host cell.

An eukaryotic host cell may be a fungal host cell. "Fungi" include all species of the subdivision *Eumycotina* (Alexopoulos, C. J., 1962, In: Introductory Mycology, John Wiley & Sons, Inc., New York). The term fungus thus includes among others filamentous fungi and yeast.

"Filamentous fungi" are herein defined as eukaryotic microorganisms that include all filamentous forms of the subdivision *Eumycotina* and *Oomycota* (as defined by Hawksworth *et al*., 1995, supra). The filamentous fungi are characterized by a mycelial wall composed of chitin, cellulose, glucan, chitosan, mannan, and other complex polysaccharides. Vegetative growth is by hyphal elongation and carbon catabolism is obligatory aerobic. Filamentous fungal strains include, but are not limited to, strains of *Acremonium, Aspergillus, Agaricus, Aureobasidium, Cryptococcus, Corynascus, Chrysosporium, Filibasidium, Fusarium, Humicola, Magnaporthe, Monascus, Mucor, Myceliophthora, Mortierella, Neocallimastix, Neurospora, Paecilomyces, Penicillium, Piromyces, Phanerochaete Podospora, Pycnoporus, Rhizopus, Schizophyllum, Sordaria, Talaromyces, Rasmsonia, Thermoascus, Thielavia, Tolypocladium, Trametes* and *Trichoderma.* Preferred filamentous fungal strains that may serve as host cells belong to the species *Aspergillus niger, Aspergillus oryzae, Aspergillus fumigatus, Penicillium chrysogenum, Penicillium citrinum, Acremonium chrysogenum, Trichoderma reesei, Rasamsonia emersonii* (formerly known as *Talaromyces emersonii*), *Aspergillus sojae, Chrysosporium lucknowense, Myceliophtora thermophyla.* Reference host cells for the comparison of fermentation characteristics of transformed and untransformed cells, include e.g. *Aspergillus niger* CBS120.49, CBS 513.88, *Aspergillus oryzae* ATCC16868, ATCC 20423, IFO 4177, ATCC 1011, ATCC 9576, ATCC14488-14491, ATCC 11601, ATCC12892, *Aspergillus fumigatus* AF293 (CBS101355), *P. chrysogenum* CBS 455.95, *Penicillium citrinum* ATCC 38065, *Penicillium chrysogenum* P2, *Acremonium chrysogenum* ATCC 36225, ATCC 48272, *Trichoderma reesei* ATCC 26921, ATCC 56765, ATCC 26921, *Aspergillus sojae* ATCC11906, *Chrysosporium lucknowense* ATCC44006 and derivatives of all of these strains. Particularly preferred as filamentous fungal host cell are *Aspergillus niger* CBS 513.88 and derivatives thereof.

An eukaryotic host cell may be a yeast cell. Preferred yeast host cells may be selected from the genera: *Saccharomyces* (e.g., *S*. *cerevisiae, S. bayanus, S. pastorianus, S. carlsbergensis*), *Brettanomyces, Kluyveromyces, Candida* (e.g., *C*. *krusei, C. revkaufi, C. pulcherrima, C. tropicalis, C. utilis*), *Issatchenkia* (eg. *I. orientalis*) *Pichia* (e.g., *P. pastoris*), *Schizosaccharomyces, Hansenula, Kloeckera, Pachysolen, Schwanniomyces, Trichosporon, Yarrowia* (e.g., *Y. lipolytica* (formerly classified as *Candida lipolytica*)), *Yamadazyma*.

Prokaryotic host cells may be bacterial host cells. Bacterial host cell may be Gram negative or Gram positive bacteria. Examples of bacteria include, but are not limited to, bacteria belonging to the genus *Bacillus* (e.g., *B*. *subtilis, B. amyloliquefaciens, B. licheniformis, B. puntis, B. megaterium, B. halodurans, B. pumilus*,), *Acinetobacter, Nocardia, Xanthobacter, Escherichia* (e.g., *E. coli* (e.g., strains DH 1 OB, Stbl2, DH5-alpha, DB3, DB3.1), DB4, DB5, JDP682 and ccdA-over (e.g., U.S. application No. 09/518,188))), *Streptomyces, Erwinia, Klebsiella, Serratia* (e.g., S. *marcessans*), *Pseudomonas* (e.g., *P*. *aeruginosa*), *Salmonella* (e.g., *S*. *typhimurium, S. typhi*). Bacteria also include, but are not limited to, photosynthetic bacteria (e.g., green non-sulfur bacteria (e.g., *Choroflexus* bacteria (e.g., *C*. *aurantiacus*), *Chloronema* (e.g., *C*. *gigateum*)), green sulfur bacteria (e.g., *Chlorobium bacteria* (e.g., *C. limicola*), *Pelodictyon* (e.g., *P. luteolum*), purple sulfur bacteria (e.g., *Chromatium* (e.g., *C*. *okenii*)), and purple non-sulfur bacteria (e.g., *Rhodospirillum* (e.g., *R. rubrum*), *Rhodobacter* (e.g. *R*. *sphaeroides, R. capsulatus*), and *Rhodomicrobium* bacteria (e.g., *R*. *vanellii*)).

Host cells may be host cells from non-microbial organisms. Examples of such cells, include, but are not limited to, insect cells (e.g., *Drosophila* (e.g., *D. melanogaster*), *Spodoptera* (e.g., *S*. *frugiperda* Sf9 or Sf21 cells) and *Trichoplusa* (e.g., High-Five cells); nematode cells (e.g., C. *elegans* cells); avian cells; amphibian cells (e.g., *Xenopus laevis* cells); reptilian cells; and mammalian cells (e.g., NIH3T3, 293, CHO, COS, VERO, C127, BHK, Per-C6, Bowes melanoma and HeLa cells).

A recombinant host according to the present invention may be able to grow on any suitable carbon source known in the art and convert it to a glycosylated diterpene, eg. a steviol glycoside. The recombinant host may be able to convert directly plant biomass, celluloses, hemicelluloses, pectines, rhamnose, galactose, fucose, maltose, maltodextrines, ribose, ribulose, or starch, starch derivatives, sucrose, lactose and glycerol. Hence, a preferred host expresses enzymes such as cellulases (endocellulases and exocellulases) and hemicellulases (e.g. endo- and exo-xylanases, arabinases) necessary for the conversion of cellulose into glucose monomers and hemicellulose into xylose and arabinose monomers, pectinases able to convert pectines into glucuronic acid and galacturonic acid or amylases to convert starch into glucose monomers. Preferably, the host is able to convert a carbon source selected from the group consisting of glucose, xylose, arabinose, sucrose, lactose and glycerol. The host cell may for instance be a eukaryotic host cell as described in WO03/062430, WO06/009434, EP1499708B1, WO2006096130 or WO04/099381.

Thus, in a further aspect, the invention also provides a process for the preparation of a glycosylated diterpene which comprises fermenting a recombinant host of the invention which is capable of producing at least one glycosylated diterpene in a suitable fermentation medium, and optionally recovering the glycosylated diterpene.

The fermentation medium used in the process for the production of a glycosylated diterpene may be any suitable fermentation medium which allows growth of a particular eukaryotic host cell. The essential elements of the fermentation medium are known to the person skilled in the art and may be adapted to the host cell selected.

Preferably, the fermentation medium comprises a carbon source selected from the group consisting of plant biomass, celluloses, hemicelluloses, pectines, rhamnose, galactose, fucose, fructose, maltose, maltodextrines, ribose, ribulose, or starch, starch derivatives, sucrose, lactose, fatty acids, triglycerides and glycerol. Preferably, the fermentation medium also comprises a nitrogen source such as ureum, or an ammonium salt such as ammonium sulphate, ammonium chloride, ammoniumnitrate or ammonium phosphate.

The fermentation process according to the present invention may be carried out in batch, fed-batch or continuous mode. A separate hydrolysis and fermentation (SHF) process or a simultaneous saccharification and fermentation (SSF) process may also be applied. A combination of these fermentation process modes may also be possible for optimal productivity. A SSF process may be particularly attractive if starch, cellulose, hemicelluose or pectin is used as a carbon source in the fermentation process, where it may be necessary to add hydrolytic enzymes, such as cellulases, hemicellulases or pectinases to hydrolyse the substrate.

The recombinant host used in the process for the preparation of a glycosylated diterpene may be any suitable recombinant host as defined herein above. It may be advantageous to use a recombinant eukaryotic recombinant host according to the invention in the process since most eukaryotic cells do not require sterile conditions for propagation and are insensitive to bacteriophage infections. In addition, eukaryotic host cells may be grown at low pH to prevent bacterial contamination.

The recombinant host according to the present invention may be a facultative anaerobic microorganism. A facultative anaerobic recombinant host can be propagated aerobically to a high cell concentration. This anaerobic phase can then be carried out at high cell density which reduces the fermentation volume required substantially, and may minimize the risk of contamination with aerobic microorganisms.

The fermentation process for the production of a glycosylated diterpene according to the present invention may be an aerobic or an anaerobic fermentation process.

An anaerobic fermentation process may be herein defined as a fermentation process run in the absence of oxygen or in which substantially no oxygen is consumed, preferably less than 5, 2.5 or 1 mmol/L/h, and wherein organic molecules serve as both electron donor and electron acceptors. The fermentation process according to the present invention may also first be run under aerobic conditions and subsequently under anaerobic conditions.

The fermentation process may also be run under oxygen-limited, or micro-aerobical, conditions. Alternatively, the fermentation process may first be run under aerobic conditions and subsequently under oxygen-limited conditions. An oxygen-limited fermentation process is a process in which the oxygen consumption is limited by the oxygen transfer from the gas to the liquid. The degree of oxygen limitation is determined by the amount and composition of the ingoing gasflow as well as the actual mixing/mass transfer properties of the fermentation equipment used.

The production of a glycosylated diterpene in the process according to the present invention may occur during the growth phase of the host cell, during the stationary (steady state) phase or during both phases. It may be possible to run the fermentation process at different temperatures.

The process for the production of a glycosylated diterpene may be run at a temperature which is optimal for the recombinant host. The optimum growth temperature may differ for each transformed recombinant host and is known to the person skilled in the art. The optimum temperature might be higher than optimal for wild type organisms to grow the organism efficiently under non-sterile conditions under minimal infection sensitivity and lowest cooling cost. Alternatively, the process may be carried out at a temperature which is not optimal for growth of the recombinant host.

The process for the production of a glycosylated diterpene according to the present invention may be carried out at any suitable pH value. If the recombinant host is a yeast, the pH in the fermentation medium preferably has a value of below 6, preferably below 5,5, preferably below 5, preferably below 4,5, preferably below 4, preferably below pH 3,5 or below pH 3,0, or below pH 2,5, preferably above pH 2. An advantage of carrying out the fermentation at these low pH values is that growth of contaminant bacteria in the fermentation medium may be prevented.

Such a process may be carried out on an industrial scale. The product of such a process is one or more glycosylated diterpenes, such as one or more steviol glycosides.

Recovery of glycosylated diterpene(s) from the fermentation medium may be performed by known methods in the art, for instance by distillation, vacuum extraction, solvent extraction, or evaporation.

In the process for the production of a glycosylated diterpene according to the invention, it may be possible to achieve a concentration of above 5 mg/l fermentation broth, preferably above 10 mg/l, preferably above 20 mg/l, preferably above 30 mg/l fermentation broth, preferably above 40 mg/l, more preferably above 50 mg/l, preferably above 60 mg/l, preferably above 70, preferably above 80 mg/l, preferably above 100 mg/l, preferably above 1 g/l, preferably above 5 g/l, preferably above 10 g/l, for example above 20g/l, but usually up to a concentration of about 200g/l, such as up to about 150g/l, such as up to about 100g/l, for example up to about 70 g/l. Such concentrations may be concentration of the total broth or of the supernatant..

The invention further provides a fermentation broth comprising a glycosylated diterpene obtainable by the process of the invention for the preparation of a glycosylated diterpene.

In the event that one or more glycosylated diterpenes is expressed within a recombinant host of the invention, such cells may need to be treated so as to release them. Preferentially, at least one glycosylated diterpene, such as a steviol glycoside, for example rebA or rebM, is produced extracellularly

The invention also provides a glycosylated diterpene obtained by a process according to the invention for the preparation of a glycosylated diterpene or obtainable from a fermentation broth of the invention. Such a glycosylated diterpene may be a non- naturally occurring glycosylated diterpene, that is to say one which is not produced in plants.

Also provided is a composition comprising one or more steviol glycosides obtainable by process for the preparation of a glycosylated diterpene or obtainable from a fermentation broth of the invention. Such a composition may comprise two or more glycosylated diterpenes obtainable by a process of the invention for the preparation of a glycosylated diterpene or obtainable from a fermentation broth of the invention. In such a composition, one or more of the glycosylated diterpenes may be a non-naturally occurring glycosylated diterpene, that is to say one which is not produced in plants.

Furthermore, the invention provides a method for converting a first glycosylated diterpene into a second glycosylated diterpene, which method comprises:
- contacting said first glycosylated diterpene with a recombinant host of the invention, a cell free extract derived from such a recombinant host or an enzyme preparation derived from either thereof;
- thereby to convert the first glycosylated diterpene into the second glycosylated diterpene.

In such a method, the second glycosylated diterpene may be steviol-19-diside, steviolbioside, stevioside, 13-[(β-D-Glucopyranosyl)oxy)kaur-16-en-18-oic acid 2-O-β-D-glucopyranosyl-p-D-glucopyranosyl ester, Reb E or Reb D.

In such a method, the first glycosylated diterpene may be steviol-13-monoside, steviol-19-monoside, rubusoside, stevioside, rebaudioside A or 13-[(β-D-Glucopyranosyl)oxy)kaur-16-en-18-oic acid 2-O-β-D-glucopyranosyl-β-D-glucopyranosyl ester and the second glycosylated diterpene is steviol-19-diside, steviolbioside, stevioside, 13-[(β-D-Glucopyranosyl)oxy)kaur-16-en-18-oic acid 2-O-β-D-glucopyranosyl-β-D-glucopyranosyl ester, Reb E or Reb D.

These are the first and second steviol glycosides in relation to a reaction catalysed by a polypeptide described herein having UGT2 activity.

That is to say, the invention relates to a method of bioconversion or biotransformation.

A steviol glycoside or composition produced by the fermentation process according to the present invention may be used in any application known for such compounds. In particular, they may for instance be used as a sweetener, for example in a food or a beverage. According to the invention therefore, there is provided a foodstuff, feed or beverage which comprises a glycosylated diterpene, such as a steviol glycoside, or a composition of the invention.

For example a glycosylated diterpene or a composition of the invention may be formulated in soft drinks, as a tabletop sweetener, chewing gum, dairy product such as yoghurt (e.g. plain yoghurt), cake, cereal or cereal-based food, nutraceutical, pharmaceutical, edible gel, confectionery product, cosmetic, toothpastes or other oral cavity composition, etc. In addition, a glycosylated diterpene or a composition of the invention can be used as a sweetener not only for drinks, foodstuffs, and other products dedicated for human consumption, but also in animal feed and fodder with improved characteristics.

Accordingly, the invention provides, *inter alia,* a foodstuff, feed or beverage which comprises a diterpene or glycosylated diterpene prepared according to a process of the invention.

During the manufacturing of foodstuffs, drinks, pharmaceuticals, cosmetics, table top products, chewing gum the conventional methods such as mixing, kneading, dissolution, pickling, permeation, percolation, sprinkling, atomizing, infusing and other methods can be used.

The glycosylated diterpene, for example a steviol glycoside, or a composition of the invention can be used in dry or liquid forms. It can be added before or after heat treatment of food products. The amount of the sweetener depends on the purpose of usage. It can be added alone or in the combination with other compounds.

Compounds produced according to the method of the invention may be blended with one or more further non-caloric or caloric sweeteners. Such blending may be used to improve flavour or temporal profile or stability. A wide range of both non-caloric and caloric sweeteners may be suitable for blending with a glycosylated diterpene or a composition of the invention. For example, non-caloric sweeteners such as mogroside, monatin, aspartame, acesulfame salts, cyclamate, sucralose, saccharin salts or erythritol. Caloric sweeteners suitable for blending with a glycosylated diterpene or a composition of the invention include sugar alcohols and carbohydrates such as sucrose, glucose, fructose and HFCS. Sweet tasting amino acids such as glycine, alanine or serine may also be used.

A glycosylated diterpene or a composition of the invention can be used in the combination with a sweetener suppressor, such as a natural sweetener suppressor. It may be combined with an umami taste enhancer, such as an amino acid or a salt thereof.

A glycosylated diterpene or a composition of the invention can be combined with a polyol or sugar alcohol, a carbohydrate, a physiologically active substance or functional ingredient (for example a carotenoid, dietary fiber, fatty acid, saponin, antioxidant, nutraceutical, flavonoid, isothiocyanate, phenol, plant sterol or stanol (phytosterols and phytostanols), a polyols, a prebiotic, a probiotic, a phytoestrogen, soy protein, sulfides/thiols, amino acids, a protein, a vitamin, a mineral, and/or a substance classified based on a health benefits, such as cardiovascular, cholesterol-reducing or anti-inflammatory.

A composition with a glycosylated diterpene or a composition of the invention may include a flavoring agent, an aroma component, a nucleotide, an organic acid, an organic acid salt, an inorganic acid, a bitter compound, a protein or protein hydrolyzate, a surfactant, a flavonoid, an astringent compound, a vitamin, a dietary fiber, an antioxidant, a fatty acid and/or a salt.

A glycosylated diterpene or a composition of the invention may be applied as a high intensity sweetener to produce zero calorie, reduced calorie or diabetic beverages and food products with improved taste characteristics. Also it can be used in drinks, foodstuffs, pharmaceuticals, and other products in which sugar cannot be used.

In addition, a glycosylated diterpene or a composition of the invention may be used as a sweetener not only for drinks, foodstuffs, and other products dedicated for human consumption, but also in animal feed and fodder with improved characteristics.

The examples of products where a glycosylated diterpene or a composition of the invention can be used as a sweetening compound can be as alcoholic beverages such as vodka, wine, beer, liquor, sake, etc.; natural juices, refreshing drinks, carbonated soft drinks, diet drinks, zero calorie drinks, reduced calorie drinks and foods, yogurt drinks, instant juices, instant coffee, powdered types of instant beverages, canned products, syrups, fermented soybean paste, soy sauce, vinegar, dressings, mayonnaise, ketchups, curry, soup, instant bouillon, powdered soy sauce, powdered vinegar, types of biscuits, rice biscuit, crackers, bread, chocolates, caramel, candy, chewing gum, jelly, pudding, preserved fruits and vegetables, fresh cream, jam, marmalade, flower paste, powdered milk, ice cream, sorbet, vegetables and fruits packed in bottles, canned and boiled beans, meat and foods boiled in sweetened sauce, agricultural vegetable food products, seafood, ham, sausage, fish ham, fish sausage, fish paste, deep fried fish products, dried seafood products, frozen food products, preserved seaweed, preserved meat, tobacco, medicinal products, and many others. In principal it can have unlimited applications.

The sweetened composition comprises a beverage, non-limiting examples of which include non-carbonated and carbonated beverages such as colas, ginger ales, root beers, ciders, fruit-flavored soft drinks (e.g., citrus-flavored soft drinks such as lemon-lime or orange), powdered soft drinks, and the like; fruit juices originating in fruits or vegetables, fruit juices including squeezed juices or the like, fruit juices containing fruit particles, fruit beverages, fruit juice beverages, beverages containing fruit juices, beverages with fruit flavorings, vegetable juices, juices containing vegetables, and mixed juices containing fruits and vegetables; sport drinks, energy drinks, near water and the like drinks (e.g., water with natural or synthetic flavorants); tea type or favorite type beverages such as coffee, cocoa, black tea, green tea, oolong tea and the like; beverages containing milk components such as milk beverages, coffee containing milk components, cafe au lait, milk tea, fruit milk beverages, drinkable yogurt, lactic acid bacteria beverages or the like; and dairy products.

Generally, the amount of sweetener present in a sweetened composition varies widely depending on the particular type of sweetened composition and its desired sweetness. Those of ordinary skill in the art can readily discern the appropriate amount of sweetener to put in the sweetened composition.

A glycosylated diterpene or a composition of the invention can be used in dry or liquid forms. It can be added before or after heat treatment of food products. The amount of the sweetener depends on the purpose of usage. It can be added alone or in the combination with other compounds.

During the manufacturing of foodstuffs, drinks, pharmaceuticals, cosmetics, table top products, chewing gum the conventional methods such as mixing, kneading, dissolution, pickling, permeation, percolation, sprinkling, atomizing, infusing and other methods can be used.

Thus, compositions of the present invention can be made by any method known to those skilled in the art that provide homogenous even or homogeneous mixtures of the ingredients. These methods include dry blending, spray drying, agglomeration, wet granulation, compaction, co-crystallization and the like.

In solid form a glycosylated diterpene or a composition of the invention can be provided to consumers in any form suitable for delivery into the comestible to be sweetened, including sachets, packets, bulk bags or boxes, cubes, tablets, mists, or dissolvable strips. The composition can be delivered as a unit dose or in bulk form.

For liquid sweetener systems and compositions convenient ranges of fluid, semi-fluid, paste and cream forms, appropriate packing using appropriate packing material in any shape or form shall be invented which is convenient to carry or dispense or store or transport any combination containing any of the above sweetener products or combination of product produced above.

The composition may include various bulking agents, functional ingredients, colorants, flavors.

The terms "sequence homology" or "sequence identity" or "homology" or "identity" are used interchangeably herein. For the purpose of this invention, it is defined here that in order to determine the percentage of sequence homology or sequence identity of two amino acid sequences or of two nucleic acid sequences, the sequences are aligned for optimal comparison purposes. In order to optimize the alignment between the two sequences gaps may be introduced in any of the two sequences that are compared. Such alignment can be carried out over the full length of the sequences being compared. Alternatively, the alignment may be carried out over a shorter length, for example over about 20, about 50, about 100 or more nucleic acids/based or amino acids. The sequence identity is the percentage of identical matches between the two sequences over the reported aligned region.

A comparison of sequences and determination of percentage of sequence identity between two sequences can be accomplished using a mathematical algorithm. The skilled person will be aware of the fact that several different computer programs are available to align two sequences and determine the identity between two sequences (Kruskal, J. B. (1983) An overview of sequence comparison In D. Sankoff and J. B. Kruskal, (ed.), Time warps, string edits and macromolecules: the theory and practice of sequence comparison, pp. 1-44 Addison Wesley). The percent sequence identity between two amino acid sequences or between two nucleotide sequences may be determined using the Needleman and Wunsch algorithm for the alignment of two sequences. (Needleman, S. B. and Wunsch, C. D. (1970) J. Mol. Biol. 48, 443-453). Both amino acid sequences and nucleotide sequences can be aligned by the algorithm. The Needleman-Wunsch algorithm has been implemented in the computer program NEEDLE. For the purpose of this invention the NEEDLE program from the EMBOSS package was used (version 2.8.0 or higher, EMBOSS: The European Molecular Biology Open Software Suite (2000) Rice,P. Longden, I. and Bleasby,A. Trends in Genetics 16, (6) pp276-277, http://emboss.bioinformatics.nl/). For protein sequences EBLOSUM62 is used for the substitution matrix. For nucleotide sequence, EDNAFULL is used. The optional parameters used are a gap-open penalty of 10 and a gap extension penalty of 0.5. The skilled person will appreciate that all these different parameters will yield slightly different results but that the overall percentage identity of two sequences is not significantly altered when using different algorithms.

After alignment by the program NEEDLE as described above the percentage of sequence identity between a query sequence and a sequence of the invention is calculated as follows: Number of corresponding positions in the alignment showing an identical amino acid or identical nucleotide in both sequences divided by the total length of the alignment after subtraction of the total number of gaps in the alignment. The identity defined as herein can be obtained from NEEDLE by using the NOBRIEF option and is labeled in the output of the program as "longest-identity".

The nucleic acid and protein sequences of the present invention can further be used as a "query sequence" to perform a search against public databases to, for example, identify other family members or related sequences. Such searches can be performed using the NBLAST and XBLAST programs (version 2.0) of Altschul, et al. (1990) J. Mol. Biol. 215:403-10. BLAST nucleotide searches can be performed with the NBLAST program, score = 100, wordlength = 12 to obtain nucleotide sequences homologous to nucleic acid molecules as described herein. BLAST protein searches can be performed with the XBLAST program, score = 50, wordlength = 3 to obtain amino acid sequences homologous to protein molecules as described herein. To obtain gapped alignments for comparison purposes, Gapped BLAST can be utilized as described in Altschul et al., (1997) Nucleic Acids Res. 25(17): 3389-3402. When utilizing BLAST and Gapped BLAST programs, the default parameters of the respective programs (e.g., XBLAST and NBLAST) can be used. See the homepage of the National Center for Biotechnology Information at http://www.ncbi.nlm.nih.gov/.

Embodiments of the invention:
1. A recombinant host comprising a recombinant nucleic acid sequence encoding a polypeptide having:
   a. the amino acid sequence set forth in SEQ ID NO: 4 or an amino acid sequence having at least about 30% sequence identity thereto;
   b. the amino acid sequence set forth in SEQ ID NO: 2 or an amino acid sequence having at least about 30% sequence identity thereto;
   c. the amino acid sequence set forth in SEQ ID NO: 3 or an amino acid sequence having at least about 30% sequence identity thereto; or
   d. the amino acid sequence set forth in SEQ ID NO: 1 or an amino acid sequence having at least about 30% sequence identity thereto.
2. A recombinant host according to embodiment 1 which is capable of producing a glycosylated diterpene, such as a steviol glycoside.
3. A recombinant host according to embodiment 1 or 2 which comprises one or more recombinant nucleotide sequence(s) encoding:
   a polypeptide having ent-copalyl pyrophosphate synthase activity;
   a polypeptide having ent-Kaurene synthase activity;
   a polypeptide having ent-Kaurene oxidase activity; and
   a polypeptide having kaurenoic acid 13-hydroxylase activity.
4. A recombinant host according to any one of the preceding embodiments, which comprises a recombinant nucleic acid sequence encoding a polypeptide having NADPH-cytochrome p450 reductase activity.
5. A recombinant host according to any one of the preceding embodiments which comprises a recombinant nucleic acid sequence encoding one or more of:
   (i) a polypeptide having UGT74G1 activity (UGT3 activity);
   (ii) a polypeptide having UGT85C2 activity (UGT1 activity); and
   (iii) a polypeptide having UGT76G1 activity (UGT4 activity).
6. A recombinant host according to any one of the preceding embodiments which comprises a recombinant nucleic acid sequence encoding an additional polypeptide having UGT2 activity.
7. A recombinant host according to any one of the preceding embodiments, wherein the host belongs to one of the genera Saccharomyces, Aspergillus, Pichia, Kluyveromyces, Candida, Hansenula, Humicola, Issatchenkia, Trichosporon, Brettanomyces, Pachysolen, Yarrowia, Yamadazyma or Escherichia.
8. A recombinant host according to embodiment 7, wherein the recombinant host is a Saccharomyces cerevisiae cell, a Yarrowia lipolytica cell, a Candida krusei cell, an Issatchenkia orientalis or an Escherichia coli cell.
9. A recombinant host according to any one of the preceding embodiments, wherein the ability of the host to produce geranylgeranyl diphosphate (GGPP) is upregulated.
10. A recombinant host according to any one of the preceding embodiments, comprising one or more recombinant nucleic acid sequence(s) encoding hydroxymethylglutaryl-CoA reductase, farnesyl-pyrophosphate synthetase and geranylgeranyl diphosphate synthase.
11. A recombinant host according to any one of the preceding embodiments which comprises a nucleic acid sequence encoding one or more of:
   a polypeptide having hydroxymethylglutaryl-CoA reductase activity;
   a polypeptide having farnesyl-pyrophosphate synthetase activity;
   a polypeptide having geranylgeranyl diphosphate synthase activity.
12. A process for the preparation of a glycosylated diterpene which comprises fermenting a recombinant host according to any one of embodiments 2 to 11 in a suitable fermentation medium, and optionally recovering the glycosylated diterpene.
13. A process according to embodiment 12 for the preparation of a glycosylated diterpene, wherein the process is carried out on an industrial scale.
14. A fermentation broth comprising a glycosylated diterpene obtainable by the process according to embodiment 12 or 13.
15. A glycosylated diterpene obtained by a process according to embodiment 12 or 13 or obtainable from a fermentation broth according to embodiment 14.
16. A composition comprising two or more glycosylated diterpenes obtained by a process according to embodiment 12 or 13 or obtainable from a fermentation broth according to embodiment 14.
17. A foodstuff, feed or beverage which comprises a glycosylated diterpene according to embodiment 15 or a composition according to embodiment 16.
18. A method for converting a first glycosylated diterpene into a second glycosylated diterpene, which method comprises:
   - contacting said first glycosylated diterpene with a recombinant host according to any one of embodiments 1 to 11, a cell free extract derived from such a recombinant host or an enzyme preparation derived from either thereof;
   - thereby to convert the first glycosylated diterpene into the second glycosylated diterpene.
19. A method according to embodiment 18, wherein the second glycosylated diterpene is: steviol-19-diside, steviolbioside, stevioside, 13-[(β-D-Glucopyranosyl)oxy)kaur-16-en-18-oic acid 2-O-β-D-glucopyranosyl-β-D-glucopyranosyl ester, Reb E or Reb D.
20. A method according to embodiment 19, wherein the first glycosylated diterpene is steviol-13-monoside, steviol-19-monoside, rubusoside, stevioside, Rebaudioside A or 13-[(β-D-Glucopyranosyl)oxy)kaur-16-en-18-oic acid 2-O-β-D-glucopyranosyl-β-D-glucopyranosyl ester and the second glycosylated diterpene is steviol-19-diside, steviolbioside, stevioside, 13-[(β-D-Glucopyranosyl)oxy)kaur-16-en-18-oic acid 2-O-β-D-glucopyranosyl-β-D-glucopyranosyl ester, Reb E or Reb D.

A reference herein to a patent document or other matter which is given as prior art is not to be taken as an admission that that document or matter was known or that the information it contains was part of the common general knowledge as at the priority date of any of the claims.

The disclosure of each reference set forth herein is incorporated herein by reference in its entirety.

The present invention is further illustrated by the following Examples:

### EXAMPLES

### Example 1: Construction of E. coli expression vectors

The full length open reading frame encoding UGTs from *Solanum lycopersicon* were amplified from *S*. *lycopersicon* cDNA. 1 µg of total RNA isolated from tomato fruit was used as starting material to prepare cDNA using the SMART^{™} RACE cDNA Amplification Kit (Clontech), according to the manufacturer's instructions.

For amplification Phusion "proofreading polymerase" (Finnzymes) and the primers mentioned in Table 1 were used.

**Table 1: primers used to amplify tomato and stevia UGT fragments**

| | **Forward primer** | **Reverse primer** |
|---|---|---|
| RT7 | ATTAGGATCCAATGGGAACACAAGTAACAGAG | AATACTGCAGTTAATTAGTACTAATCTTACAAAATTG |
| RT11 | ATTAGGATCCAATGGAAGCCAAGAAAAATAAAATGAG | AATACTGCAGTCATTTGTTGCTGCAAAGAGCCATC |
| RT15 | ATTAGGATCCAATGGATGGTTCGAATGAAAAGTC | AATACTGCAGCTAGACAACATTTGATCTAGTCTTG |
| RT18 | ATTAGGATCCAATGAGTACTACTTTAAAGGTATTGATG | AATACTGCAGATTCACTTATTACTATTCCTACAAAG |
| UGT2_1 a | ATTAGGATCCAATGGCCACTTCTGACTCCAT | AATAAAGCTTTTAGCTTTCGTGGTCAATGGCA |
| 85C2 | ATTAGGATCCAATGGACGCTATGGCCACCACT | AATAAAGCTTTTAGTTTCGAGCCAAGACAGTG |

The amplified fragment and vector pACYC-DUET1 (Novagen) were digested with the restriction enzymes *Bam*HI and *Pst*I for the tomato UGT fragments or *Bam*HI and *Hind*III for UGT2_1 a and UGT85C2, followed by purification of the required DNA fragments, their subsequent ligation and finally transformation into *E*. *coli* XL-1 blue using standard procedures. Recombinant bacteria were selected on LB plates containing 50 µg /mL chloramphenicol. After ON growth of recombinant colonies in liquid culture (3 mL LB broth with 50 µg /mL chloramphenicol, 250 rpm, 37°C), plasmid DNA was isolated using the Qiaprep Spin Miniprep kit (Qiagen). Isolated plasmid material was checked by Sanger sequencing with vector primers.

This cloning strategy led to constructs from which the UGTs can be expressed with an N-terminal His₆-tag

### Example 2: Synthesis of Steviolmonoside by UGT85C2

To prepare Steviolmonoside enzymatically from Steviol (Sigma U4625) and UDP-glucose (Sigma 4625), the following compounds were mixed in a total reaction volume of 4 ml. For preparation of a crude enzyme extract of UGT85C2 see Example 3.

| | **µl** |
|---|---|
| 100 mM 2-mercaptoethanol in 0.1 M Tris-buffer | 160 |
| 100 mM UDP-glucose in 10% DMSO | 800 |
| 100 mM Steviol in 100% DMSO | 40 |
| Crude enzyme extract UGT85C2 | 400 |

The glycosylation reaction was performed overnight at 30 °C and 100 rpm.
Subsequently the reaction was purified using an Oasis hydrophiliclipophilic-Balanced (HLB) 3 cc extraction cartridge (Waters), which had been preconditioned according to the manufacturer's instructions. The enzymatic reaction was loaded on the HLB column, and allowed to enter the column by gravity flow. Subsequently the column was washed with 6 mL of water. Product was eluted by passing 3 ml of 100% methanol over the column. The methanol elute was dried under vacuum centrifugation and the pellet dissolved in 80 µl DMSO. This resulted in a 50 mM steviolmonoside preparation.

### Example 3: In vitro comparison of different tomato UGTs and Stevia UGT2 1a

The control plasmid pACYC-DUET-1 and the UGT constructs were transformed to *E. coli* BL21 DE3 (Invitrogen). For expression, a 3 mL overnight culture of the recombinant *E*. *coli* strains was prepared (LB medium with appropriate antibiotic; 50 ug chloramphenicol / mL and 1% glucose). 200 µL of that culture was transferred to 20 mL of LB medium with the appropriate antibiotic in a 100 mL Erlenmeijer flask, and incubated at 37°C, 250 rpm until the A600 was 0.4 to 0.6. Subsequently IPTG was added to a final concentration of 1 mM and cultures were incubated overnight at 18°C and 250 rpm. The next day, cells were harvested by centrifugation (10 min 8000xg), medium was removed, and cells were resuspended in 1 mL Resuspension buffer (100 mM Tris-HCI pH = 7.5, 1.4 mM 2-mercaptoethanol; 4°C, 15% glycerol). Cells were disrupted by two times shaking with 200 mg 0.1 mm Zirconia/Silica Beads (BioSpec) for 10 seconds in a FastPrep FP120 machine (Savant) at speed 6.5 . Insoluble particles were subsequently removed by centrifugation (10 min 13,000xg, 4°C). The resulting supernatants were referred to as crude enzyme extracts.

### Example 4: Glucosylation of Steviolmonoside and RebaudiosideA by UGTs

For enzyme assays, a mix of total 50 µl was made in a 2 ml eppendorf tube:

| | |
|---|---|
| 0.1 M Tris in 2% DMSO | 37.5µl |
| 100 mM 2-mercaptoethanol in 0.1M tris | 2 µl |
| 100 mM UDP-glucose in 10%DMSO | 5 µl |
| 50 mM Steviolmonoside in 100%DMSO | 0.5 µl |
| Crude UGT enzyme extract | 5 µl |

The tubes were incubated overnight at 30 °C and 100 rpm.

For assays with Rebaudiosise-A (Reb A), Steviolmonoside was replaced by 0.5 µl 50mM Reb A (ChromDex ASB-00018225) in 50% DMSO.

### Example 5: LC-MS analyses

An LC-PDA-QTOF-MS system was used to analyse reaction products. After incubation, the *in vitro* enzyme assay mix (50 µl) was stopped by addition of 150 µl of 100% methanol in MQ water acidified with 0.13% formic acid. Samples were sonicated for 15 min, centrifuged at 2500 rpm for 10 min and filtered through 0.45µm filters (Minisart SRP4, Biotech GmbH, Germany). For chromatographic separation, a Luna C18(2) pre-column (2.0 x 4 mm) and an analytical column (2.0 x 150 mm, 100Å, particle size 3µm) from Phenomenex (Torrance, CA, USA) were used. Five microliters of each filtered sample were injected into the system for LC-PDA-MS analysis using formic acid : water (1:1000, v/v; eluent A) and formic acid : acetonitrile (1:1000, v/v; eluent B) as elution solvents. Flow was set at 0.19 mL/min with the gradient from 80% eluent A and 20% eluent B to 45% Eluent A and 55% eluent B across a period of 45 min. The column temperature was maintained at 40°C and the samples at 20°C. UV absorbance was measured using a Waters 2996 PDA (A range from 240 to 600 nm) and ESI-MS analysis was performed using a QTOF Ultima V4.00.00 mass spectrometer (Waters-Corporation, MS technologies) in negative mode. A collision energy of 10 eV was used for full-scan LC-MS in the m/z range 100 to 1,500. Leucine enkephalin ([M - H]⁻ = 554.2620), was used for online mass calibration (lock mass).

Compounds were identified by their retention time and their apparent mass and compared to standard steviosides present in the Rebaudioside-A Impurities Mix-6 (Cerilliant S-017) (Table 2).

**Table 2: Retention time and masses of steviosides in the Rebaudioside-A Impurities Mix-6**

| | **Rt (min)** | **m/z** |
|---|---|---|
| Reb D | 12.95 | 1127.47 |
| Reb A | 18.76 | 965.42 |
| Stevioside | 18.94 | 803.37 |
| Rubusoside | 22.84 | 803.37 |
| Reb B | 25.15 | 641.31 |
| Steviolbioside | 25.58 | 641.31 |
| Steviol | 44.73 | 317.21 |

The results of the *in vitro* tests are given in Table 3 and 4. To semi-quantify the produced compounds of the *in vitro* assays, the peak surface area for each relevant peak was measured from the total ion count chromatograms. Clearly, UGT2_1a was able to produce steviolbioside (Rt = 25.6) from Steviolmonoside. UGT RT18 also produces predominantly steviolbioside. Other RTs produce preferentially other steviolglycosides (Table 3).

**Table 3: Products detected by LC-MS after in vitro reaction of Steviolmonoside with different UGT enzymes. As substrate, Steviolmonoside (Rt=30.4 min; m/z 959 =[2M-H]) was used. Shown are peak surface area in the LC-MS chromatograms. Rt: retention time in minutes. Steviolbioside is detected at 25.6 min.**

| | m/z=1011 | m/z=407 | m/z = 803 | m/z = 803 | m/z = 641 | m/z = 641 | m/z = 641 |
|---|---|---|---|---|---|---|---|
| | Rt=14.0 | Rt=19.0 | Rt=20.7 | Rt=20.8 | Rt=24.8 | Rt=25.6 | Rt=26.3 |
| blanc | | | | | | 2 | |
| UGT2_1 a | | 23 | | | | 11532 | |
| RT18 | | | | | 153 | 4408 | |
| RT15 | | | 540 | | 5841 | 8467 | 1 |
| RT11 | 3 | | | 10110 | 16229 | 2163 | |
| RT7 | | | | 163 | 7764 | 1788 | |

When testing Reb A as a substrate, it was clear that RT18 showed a relatively strong formation of Reb D (Rt=12.9 min) from Reb A, in comparison with UGT2_1 a, while the other UGTs preferentially produce different Reb A-glycosides (Table 4).

**Table 4: Products detected by LC-MS after in vitro reaction of Reb A with different UGT enzymes. As substrate, Reb A (Rt=18.74 min; m/z 1011 =[M-H + formic acid]) was used. Shown are peak surface area in the LC-MS chromatograms. Rt: retention time in minutes. Reb D is detected at Rt=12.9 min.**

| | m/z=565 | m/z=1127 | m/z=1127 | m/z=1127 | m/z=1127 | m/z=1127 |
|---|---|---|---|---|---|---|
| | Rt=12.6 | Rt=12.9 | Rt=14.1 | Rt=14.25 | Rt=14.9 | Rt=17.4 |
| Blanc | | 6 | | 12 | | |
| UGT2_1 a | | 1037 | 16 | | | |
| RT18 | 98 | 3596 | | 494 | | |
| RT15 | | 376 | | 8946 | 234 | |
| RT11 | | 33 | | 7684 | 159 | |
| RT7 | | 1212 | | 2169 | | 31 |

Thus, RT18 can form steviolbioside from steviolmonoside, and Reb D from Reb A.

### Example 6: UGT protein content in crude-enzyme extracts

We observed that the activity for the formation of steviolbioside of the RT18 crude enzyme extract was 2-3 fold lower compared to UGT2_1 a. To be able to compare the two enzymes for the activity per enzyme molecule in the crude extracts, we analysed the total protein content of the crude enzyme extracts.

First, the extracts were compared for protein content using Protein Dye Reagent concentrate (BIO-RAD 500-0006), according to the manufacturer's instructions, using lyophilized Bovine Serum Albumine BioRad 500-0007) as a standard. Based on this it was observed that UGT2_1 a crude extract contained twice as much protein as the RT18 crude extract (Table 5).

**Table 5: Total protein content of crude enzyme extracts. Protein concentration is given in µq/µl**

| | **Total protein (µg/µl)** |
|---|---|
| pACYC-DUET-1 | 3.35 |
| UGT2_1a | 5.08 |
| RT18 | 2.66 |
| RT15 | 3.63 |
| RT11 | 3.33 |
| RT7 | 2.06 |

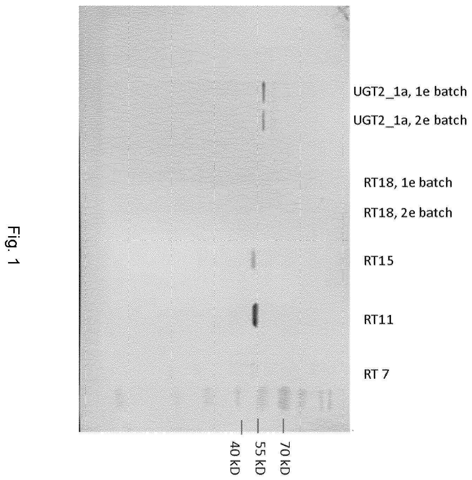

Subsequently, to compare the enzyme concentrations in the crude extracts, a western blot experiment was performed. 50 µg of total protein was brought in 50 ul Sample buffer (20 mM Tris pH 6.8, 6% glycerol, 0.4% SDS, 20 mM Dithiothreitol, 0.01% Bromophenol Blue) and boiled for 5 minutes. Subsequently 10 µl sample (= 10 µg total protein) was loaded on a 12.5 % poly-acryl amide gel with SDS and run for 2 hours at 20 mA. Proteins were transferred from the gel onto nitrocellulose membrane (BIO-RAD) in standard blotting buffer (3 g/L Tris, 14.4 g/L glycine, 10% ethanol) for 1 hour (100 V). The nitrocellulose was subsequently washed with TBST buffer (20 mM Tris-CI buffer pH 7.5, 150 mM NaCl, 0.05% Tween 20) for 5 minutes, and blocked with TBST buffer with 2% non-fat dry milk powder (ELK) for 1 hour. The presence of enzyme was detected by incubation for 1 hour with TBST with 2% ELK and 1:4000 diluted antiHis monoclonal antibody conjugated to peroxidase (Sigma, St Louis, A7058). After washing four times five minutes with TBST, the peroxidase was detected by the TMB Liquid substrate system for membranes (Sigma T0565). A purple colour was detected at the position where His-tagged proteins (here: UGTs) were present on the blot. When all five crude enzyme extracts were compared in this way (Fig. 1) it was clear that UGT2_1a was expressed to well-detectable levels, while RT18 protein could not be detected. The other UGTs (RT15, RT11,RT7) were also detected, to different intensities.

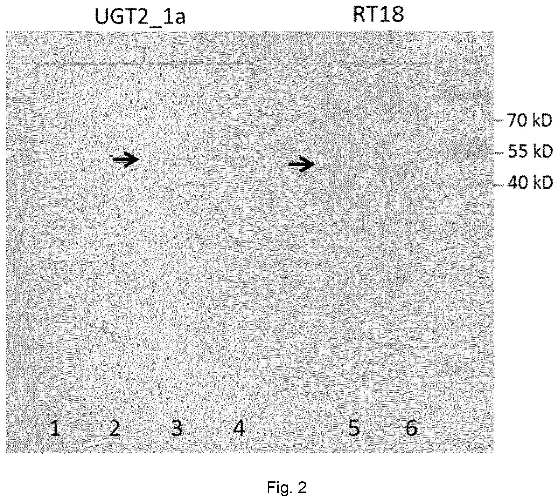

To compare the UGT content in the crude enzyme extracts of RT18 and UGT2_1a, another western blot was made. For UGT2_1a, 0.5, 1.0, 1.9, 3.8 µg protein was loaded, while for RT18, 31.9 and 63.8 µg was loaded. Detection of UGTs was performed as described above. The blot (Fig. 2) showed that the amount of His-tagged UGT protein was the same in 1.9 µg UGT2_1a extract and 63.8 µg RT18, as estimated by visual inspection. This indicated that the concentration of UGT protein in the RT18 crude extract was 20-50 fold lower than in the UGT2_1a crude extract. Thus, the activity of RT18, as recorded in Tables 3 and 4, is more than 10-fold higher than UGT2_1a when using steviolmonoside as a substrate, and more than 50-fold higher when using Reb A as a substrate.

### Example 7: MSMS analysis

To provide more evidence that the products of UGT2_1a and RT18 with steviolmonoside as a substrate were identical, the steviolbioside product of RT18 was further compared to the steviolbioside product from UGT2_1a and the steviol-diglucoside product from RT11 by tandem mass spectrometry analysis (LC-MS²). The methanol extracts from the RT18 and UGT2_1a enzyme assays were injected in an Accela HPLC-PDA (Thermo) coupled to a LTQ Ion Trap-Orbitrap FTMS hybrid mass spectrometer (Thermo) system was used. Data-directed MSMS was performed using the same LC conditions as described for LC-QTOF MS analysis (see above), and using negative ionization mode, with an Isolation Width of 3.00 Dalton and a Normalized Collission Energy of 35.0. Retention times of steviolglucosides differed slightly from the analysis on the LC-QTOF MS system (see above, Table 3).

Fragmentation was performed on the compounds with m/z 641.30 eluting at 23.0 min in the RT18 and UGT2_1a samples and eluting at 22.2 min in the RT11 sample.

In the fragmentation spectra of ions of m/z 641.30, the fragments m/z 479.26 [M-H-Glucose] and m/z 317.21 [M-H-2Glucose] were observed in all three samples. The ratio between the m/z 317.21 and m/z 479.26 ions was recorded for all three compounds. For both the RT18 and UGT2_1a compounds, the ratio m/z 317 to m/z 479 was 2:10, while the ratio m/z 317 to m/z 479 for the RT11 compound was 4:10. Thus, the MS2 analysis did not differentiate the steviolbioside products from RT18 and UGT2_1a, but did differentiate the RT11 steviol diglucoside product from these two. These results further confirm that the major product of RT18 corresponds to steviolbioside.

### Example 8. RT18 expression in steviol glycoside production strain

In order to demonstrate the effect of the in vivo activity of the RT18 enzyme on the production of steviol glycosides, RT18 (SEQ ID NO: 19) was assembled with three promoters of different strength (Table 6), and transformed to a *Yarrowia lipolytica* strain that produces steviol glycosides using the approach described in WO2013/110673 and WO2015/007748. The genotype of this Yarrowia strain is given in Table 7

**Table 6. Different strength promotors used for RT18 expression**

| **Relative promoters strength** | **Name** |
|---|---|
| Weak | CWP (SEQ ID NO: 20) |
| Medium | SCP2 (SEQ ID NO: 21) |
| Strong | HSP (SEQ ID NO: 22) |

**Table 7. Genotype of parental strain (copy number; SEQ ID NO).**

| | |
|---|---|
| Parent strain genotype | MATB tHMG (2; SEQ ID NO: 23) GGS (3; SEQ ID NO: 24) CPS (5; SEQ ID NO: 25) KS (4; SEQ ID NO: 26) KO (3; SEQ ID NO: 27) KAH4 (4; SEQ ID NO: 28) CPR (2; SEQ ID NO: 29) UGT1 (3; SEQ ID NO: 30) UGT2 (2; SEQ ID NO: 31) UGT3 (2; SEQ ID NO: 32) UGT4 (3; SEQ ID NO: 33) |

For positive transformants, a pre-culture was inoculated with colony material from YEPh-D agar. The pre-culture was grown in 200 µl YEP with glucose as carbon source. The pre-culture was incubated 72 hours in an Infors incubator at 30°C, 750 rpm and 80% humidity. 40 µl of pre-culture was used to inoculate 2.5 ml main culture. The main cultures were incubated 120 hours in an Infors incubator at 30°C, 550 rpm, 80% humidity. After 120 h the main culture was spun down at 2750 rpm for 10 min. Supernatant was diluted with water and acetonitrile, and measured using LC/MS.

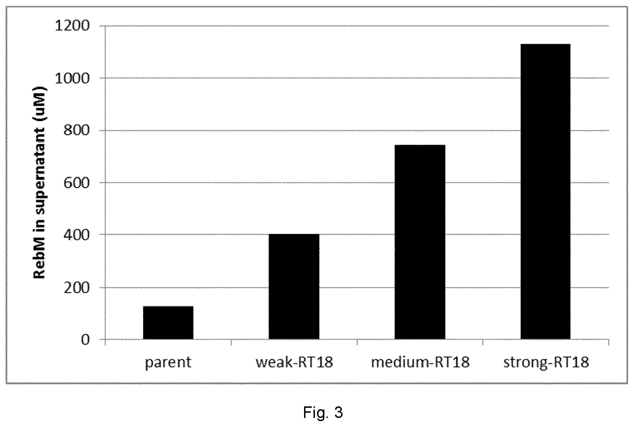

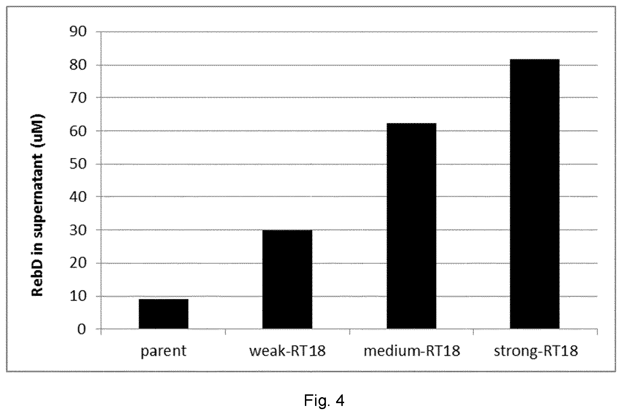

The results are set out in in Figures 3 and 4. It can be seen that the strains that express the RT18 produce higher amounts of Reb M and Reb D compared to the parent. In addition, the stronger the expression, the more Reb D and Reb M were produced. The formation of higher Reb D illustrates RT18 is effective in catalyzing the glycosylation of the glucose on the 19-position of steviol glycosides (see Figure 6), for example catalyzing the formation of Reb D from Reb A. Reb D can then be further converted to Reb M, catalyzed by UGT4.

### Example 9. RT18 and UGT4 expression in steviol glycoside production strain

The expression of other UDP-glycosyl transferases, in combination with RT18, will have an influence on the product profile. For example the Reb D that is over-produced in a strain expressing RT18 can be further converted to Reb M by the activity of UGT4. In order to evaluate the effect of over-expression of RT18 with UGT4, expression vectors of RT18 and UGT4 were transformed to a *Yarrowia lipolytica* strain producing steviol glycosides using the approach described in WO2013/110673 and WO2015/007748. The genotype of this parental strain is given in Table 8.

**Table 8. Genotype of strain used to transform RT18 and UGT4 (copy number; SEQ ID NO)**

| | |
|---|---|
| Parent strain genotype | MATB tHMG (2; SEQ ID NO: 23) GGS (2; SEQ ID NO: 24) CPS (2; SEQ ID NO: 25) KS (2; SEQ ID NO: 26) KO (2; SEQ ID NO: 27) KAH4 (2; SEQ ID NO: 28) CPR (2; SEQ ID NO: 29) UGT1 (2; SEQ ID NO: 30) UGT2 (1; SEQ ID NO: 34) UGT3 (2; SEQ ID NO: 32) UGT4 (2; SEQ ID NO: 33) |

For positive transformants, a pre-culture was inoculated with colony material from YEPh-D agar. The pre-culture was grown in 200 µl YEP with glucose as carbon source. The pre-culture was incubated 72 hours in an Infors incubator at 30°C, 750 rpm and 80% humidity. 40 µl of pre-culture was used to inoculate 2.5 ml main culture. The main cultures were incubated 120 hours in an Infors incubator at 30°C, 550 rpm, 80% humidity. After 120 h the main culture was spun down at 2750 rpm for 10 min. Supernatant was diluted with water and acetonitrile, and measured using LC/MS.

The results are set out in Table 9, where the percentages of steviol glycosides on total steviol glycosides are listed for the two strains. It can be seen that the strains that expresses the RT18 in combination with additional UGT4 effectively convert a higher percentage of the steviol glycosides to higher glycosylated steviol glycosides. Particularly, Reb B, Stevioside and Reb A are lower in the strain expressing the RT18 and UGT4, whereas the abundance of Reb M is greatly increased. This illustrates the effectiveness of RT18 expression in steering steviol glycoside production towards higher glycosylated products such as Reb M.

**Table 9. Percentages of steviol glycosides of total steviol glycosides in parent strain and strain expressing RT18 and an extra copy of UGT4.**

| **Strain** | **Reb M** | **Reb D** | **Reb A** | **Stevioside** | **Reb B** | **Other steviol glycosides** |
|---|---|---|---|---|---|---|
| parent | 3 | 6 | 54 | 25 | 7 | 6 |
| RT18, UGT4 | 66 | 5 | 20 | 1 | 1 | 6 |

**Table 10: Description of the sequence listing**

| **SEQ ID NO** | **Description** |
|---|---|
| SEQ ID NO: 1 | amino acid sequence of the RT7 protein from *Solanum lycopersicon* (Solyc11g007480 - tomato genome: http://solgenomics.net/) |
| SEQ ID NO: 2 | amino acid sequence of the RT11 protein from *Solanum lycopersicon* (Solyc11g007500) |
| SEQ ID NO: 3 | amino acid sequence of the RT15 protein from *Solanum lycopersicon* (Solyc04g081830) |
| SEQ ID NO: 4 | amino acid sequence of the RT18 protein from *Solanum lycopersicon* (Solyc05g005930) |
| SEQ ID NO: 5 | amino acid sequence of the UGT85C2 protein from *Stevia rebaudiana* |
| SEQ ID NO: 6 | amino acid sequence of the UGT2_1 a protein from *Stevia rebaudiana* |
| SEQ ID NO: 7 | nucleic acid sequence of the RT7 forward primer |
| SEQ ID NO: 8 | nucleic acid sequence of the RT7 reverse primer |
| SEQ ID NO: 9 | nucleic acid sequence of the RT11 forward primer |
| SEQ ID NO: 10 | nucleic acid sequence of the RT11 reverse primer |
| SEQ ID NO: 11 | nucleic acid sequence of the RT15 forward primer |
| SEQ ID NO: 12 | nucleic acid sequence of the RT15 reverse primer |
| SEQ ID NO: 13 | nucleic acid sequence of the RT18 forward primer |
| SEQ ID NO: 14 | nucleic acid sequence of the RT18 reverse primer |
| SEQ ID NO: 15 | nucleic acid sequence of the UGT2_1 a forward primer |
| SEQ ID NO: 16 | nucleic acid sequence of the UGT2_1 a reverse primer |
| SEQ ID NO: 17 | nucleic acid sequence of the UGT85C2 forward primer |
| SEQ ID NO: 18 | nucleic acid sequence of the UGT82C2 reverse primer |
| SEQ ID NO: 19 | nucleic acid sequence of the RT18 open reading frame optimized for expression in *Y*. *lipolytica* |
| SEQ ID NO: 20 | nucleic acid sequence of CWP promoter from *Y*. *lipolytica* |
| SEQ ID NO: 21 | nucleic acid sequence of SCP2 promoter from *Y*. *lipolytica* |
| SEQ ID NO: 22 | nucleic acid sequence of HSP promoter from *Y*. *lipolytica* |
| SEQ ID NO: 23 | nucleic acid sequence of tHMG optimized for expression in *Y*. *lipolítica* |
| SEQ ID NO: 24 | nucleic acid sequence of GGS optimized for expression in *Y*. *lipolytica* |
| SEQ ID NO: 25 | nucleic sequence of CPS from *S*. *rebaudiana* optimized for expression in *Y. lipolytica* |
| SEQ ID NO: 26 | nucleic acid sequence of tKS from *S*. rebaudiana optimized for expression in *Y. lipolytica* |
| SEQ ID NO: 27 | nucleic acid sequence of KO from *Gibberella fujikori* optimized for expression in *Y. lipolytica* |
| SEQ ID NO: 28 | nucleic acid sequence of KAH_4 optimized for expression in *Y. lipolytica* |
| SEQ ID NO: 29 | nucleic acid sequence of CPR_optimized for expression in *Y. lipolytica* |
| SEQ ID NO: 30 | nucleic acid sequence of UGT1 optimized for expression in *Y. lipolytica* |
| SEQ ID NO: 31 | nucleic acid sequence of UGT2 variant optimized for expression in *Y*. *lipolytica* |
| SEQ ID NO: 32 | nucleic acid sequence of UGT3 optimized for expression in *Y. lipolytica* |
| SEQ ID NO: 33 | nucleic acid sequence of UGT4 optimized for expression in *Y. lipolytica* |
| SEQ ID NO: 34 | nucleic acid sequence of UGT2 variant optimized for expression in *Y*. *lipolytica* |

## Claims

1. A recombinant host capable of producing glycosylated diterpene comprising a recombinant nucleic acid sequence encoding a polypeptide comprising:
a. the amino acid sequence set forth in SEQ ID NO: 4 or an amino acid sequence having at least about 30% sequence identity thereto;
b. the amino acid sequence set forth in SEQ ID NO: 2 or an amino acid sequence having at least about 30% sequence identity thereto;
c. the amino acid sequence set forth in SEQ ID NO: 3 or an amino acid sequence having at least about 30% sequence identity thereto; or
d. the amino acid sequence set forth in SEQ ID NO: 1 or an amino acid sequence having at least about 30% sequence identity thereto.

2. A recombinant host according to claim 1 which comprises one or more recombinant nucleotide sequence(s) encoding:
a polypeptide having ent-copalyl pyrophosphate synthase activity;
a polypeptide having ent-Kaurene synthase activity;
a polypeptide having ent-Kaurene oxidase activity; and
a polypeptide having kaurenoic acid 13-hydroxylase activity.

3. A recombinant host according to any one of the preceding claims, which comprises a recombinant nucleic acid sequence encoding a polypeptide having NADPH-cytochrome p450 reductase activity.

4. A recombinant host according to any one of the preceding claims which comprises a recombinant nucleic acid sequence encoding one or more of:
(i) a polypeptide having UGT74G1 activity (UGT3 activity);
(ii) a polypeptide having UGT85C2 activity (UGT1 activity); and
(iii) a polypeptide having UGT76G1 activity (UGT4 activity).

5. A recombinant host according to any one of the preceding claims which comprises a recombinant nucleic acid sequence encoding an additional polypeptide having UGT2 activity.

6. A recombinant host according to any one of the preceding claims, wherein the host belongs to one of the genera Saccharomyces, Aspergillus, Pichia, Kluyveromyces, Candida, Hansenula, Humicola, Issatchenkia, Trichosporon, Brettanomyces, Pachysolen, Yarrowia, Yamadazyma or Escherichia.

7. A recombinant host according to claim 6, wherein the recombinant host is a Saccharomyces cerevisiae cell, a *Yarrowia lipolytica* cell, a *Candida krusei* cell, an *Issatchenkia orientalis* or an *Escherichia coli* cell.

8. A recombinant host according to any one of the preceding claims, wherein the ability of the host to produce geranylgeranyl diphosphate (GGPP) is upregulated.

9. A recombinant host according to any one of the preceding claims, comprising one or more recombinant nucleic acid sequence(s) encoding hydroxymethylglutaryl-CoA reductase, farnesyl-pyrophosphate synthetase and geranylgeranyl diphosphate synthase.

10. A recombinant host according to any one of the preceding claims which comprises a nucleic acid sequence encoding one or more of:
a polypeptide having hydroxymethylglutaryl-CoA reductase activity;
a polypeptide having farnesyl-pyrophosphate synthetase activity;
a polypeptide having geranylgeranyl diphosphate synthase activity.

11. A process for the preparation of a glycosylated diterpene which comprises fermenting a recombinant host according to any one of claims 2 to 10 in a suitable fermentation medium, and optionally recovering the glycosylated diterpene.

12. A method for converting a first glycosylated diterpene into a second glycosylated diterpene, which method comprises:
- contacting said first glycosylated diterpene with a recombinant host according to any one of claims 1 to 10, a cell free extract derived from such a recombinant host or an enzyme preparation derived from either thereof;
- thereby to convert the first glycosylated diterpene into the second glycosylated diterpene.

13. A method according to claim 12, wherein the second glycosylated diterpene is: steviol-19-diside, steviolbioside, stevioside, 13-[(β-D-Glucopyranosyl)oxy)kaur-16-en-18-oic acid 2-O-β-D-glucopyranosyl-β-D-glucopyranosyl ester, Reb E or Reb D.

14. A method according to claim 13, wherein the first glycosylated diterpene is steviol-13-monoside, steviol-19-monoside, rubusoside, stevioside, Rebaudioside A or 13-[(β-D-Glucopyranosyl)oxy)kaur-16-en-18-oic acid 2-O-β-D-glucopyranosyl-β-D-glucopyranosyl ester and the second glycosylated diterpene is steviol-19-diside, steviolbioside, stevioside, 13-[(β-D-Glucopyranosyl)oxy)kaur-16-en-18-oic acid 2-O-β-D-glucopyranosyl-β-D-glucopyranosyl ester, Reb E or Reb D.


SEQUENCE LISTING

<110>  DSM IP Assets B.V.

<120>  UDP-glycosyltransferases

<130>  30028-EP-ETD

<150>  US62/136759
<151>  2015-03-23

<150>  EP16711641.7
<151>  2016-03-23

<160>  34

<170>  PatentIn version 3.5

<210>  1
<211>  453
<212>  PRT
<213>  Solanum lycopersicon

<400>  1

Met Gly Thr Gln Val Thr Glu His Gly Thr Ser Asn Leu Arg Val Val
1               5                   10                  15

Met Phe Pro Trp Leu Ala Tyr Gly His Ile Ser Pro Phe Leu Tyr Val
            20                  25                  30

Ala Lys Lys Leu Ala Asp Arg Gly Phe Leu Ile Tyr Leu Cys Ser Thr
        35                  40                  45

Pro Ile Asn Leu Lys Ser Thr Ile Glu Lys Ile Pro Glu Lys Tyr Ala
    50                  55                  60

Asp Ser Ile His Leu Ile Glu Leu His Leu Pro Glu Leu Pro Glu Leu
65                  70                  75                  80

Pro Pro His Tyr His Thr Thr Asn Gly Leu Pro Pro His Leu Asn His
                85                  90                  95

Thr Leu Gln Lys Ala Leu Lys Met Ser Lys Pro Asn Leu Ser Lys Ile
            100                 105                 110

Leu Lys Asn Leu Lys Pro Asp Leu Met Ile Tyr Asp Val Leu Gln Gln
        115                 120                 125

Trp Ala Glu Arg Val Ala Asn Glu Gln Ser Ile Pro Ala Val Arg Leu
    130                 135                 140

Leu Thr Phe Gly Ala Ala Val Phe Ser Tyr Phe Cys Asn Leu Val Lys
145                 150                 155                 160

Lys Pro Gly Val Glu Phe Pro Phe Pro Asp Ile Tyr Leu Arg Lys Ile
                165                 170                 175

Glu Gln Val Lys Leu Gly Glu Met Leu Glu Lys Ser Ala Lys Asp Gln
            180                 185                 190

Asp Pro Asp Asp Glu Glu Arg Leu Val Asp Glu Tyr Lys Gln Ile Ala
        195                 200                 205

Leu Ile Cys Thr Ser Arg Thr Ile Glu Ala Lys Tyr Ile Asp Phe Leu
    210                 215                 220

Leu Glu Leu Ser Asn Leu Lys Val Val Pro Val Gly Ser Pro Val Gln
225                 230                 235                 240

Asp Leu Ile Thr Asn Asp Ala Asp Asp Met Glu Leu Ile Asp Trp Leu
                245                 250                 255

Gly Ser Lys Asp Glu Asn Ser Thr Val Phe Val Ser Phe Gly Ser Glu
            260                 265                 270

Tyr Phe Leu Ser Lys Glu Asp Met Glu Glu Val Ala Leu Gly Leu Glu
        275                 280                 285

Leu Ser Asn Val Asn Phe Val Trp Val Ala Arg Phe Pro Lys Gly Glu
    290                 295                 300

Glu Gln Asn Leu Glu Asp Ala Leu Pro Lys Gly Phe Leu Glu Arg Ile
305                 310                 315                 320

Gly Glu Arg Gly Arg Val Leu Asp Lys Phe Ala Pro Gln Leu Arg Ile
                325                 330                 335

Leu Asn His Thr Ser Thr Gly Gly Phe Ile Ser His Cys Gly Trp Asn
            340                 345                 350

Ser Val Met Glu Ser Ile His Phe Gly Val Pro Ile Val Ala Met Pro
        355                 360                 365

Met His Leu Asp Gln Pro Met Asn Ala Arg Leu Ile Val Glu Leu Gly
    370                 375                 380

Val Ala Val Glu Ile Val Arg Asp Asp Asp Gly Lys Ile Tyr Arg Glu
385                 390                 395                 400

Glu Ile Ala Lys Thr Leu Lys Asp Val Ile Thr Glu Arg Ile Gly Glu

405                 410                 415

Asn Leu Arg Ala Lys Met Arg Glu Ile Ser Lys Asn Leu Asn Ser Ile
            420                 425                 430

Ser Gly Glu Glu Met Asp Ala Ala Ala His Glu Leu Ile Gln Phe Cys
        435                 440                 445

Lys Ile Ser Thr Asn
    450

<210>  2
<211>  444
<212>  PRT
<213>  Solanum lycopersicon

<400>  2

Met Glu Ala Lys Lys Asn Lys Met Ser Ile Leu Met Leu Pro Trp Leu
1               5                   10                  15

Ala His Gly His Ile Ser Pro Phe Leu Glu Leu Ala Lys Lys Leu Thr
            20                  25                  30

Asn Arg Asn Phe His Ile Tyr Met Cys Ser Thr Pro Ile Asn Leu Ser
        35                  40                  45

Ser Ile Lys Lys Asn Ile Thr Lys Lys Tyr Phe Glu Ser Ile Glu Leu
    50                  55                  60

Val Glu Phe His Leu Pro Ser Leu Pro Asn Leu Pro Pro His Tyr His
65                  70                  75                  80

Thr Thr Asn Gly Leu Pro Pro His Leu Met Asn Thr Leu Lys Thr Ala
                85                  90                  95

Phe Glu Asn Ala Ser Pro Asn Phe Ser Lys Ile Leu Gln Thr Leu Asn
            100                 105                 110

Pro Asp Leu Val Ile Tyr Asp Phe Asn Gln Pro Trp Ala Ala Glu Ser
        115                 120                 125

Ala Ser Ser Val Asn Ile Pro Ala Val Gln Phe Leu Thr Phe Gly Ala
    130                 135                 140

Ala Val Val Ser Leu Ala Ile His Met Phe Glu Asp Thr Glu Asp Lys
145                 150                 155                 160

Phe Pro Phe Pro Glu Ile Tyr Leu His Glu Tyr Glu Met Leu Ser Leu

                165                 170                 175

Lys Glu Ala Val Lys Glu Ala Pro Gly Asn Lys Tyr Ser Phe Asp Glu
            180                 185                 190

Ala Ile Arg Leu Ser Arg Asp Ile Val Leu Val Lys Thr Cys Arg Asp
        195                 200                 205

Phe Glu Gly Lys Tyr Val Asp Tyr Leu Ser Asn Leu Val Ser Lys Lys
    210                 215                 220

Ile Val Pro Val Gly Ser Leu Val Gln Glu Ser Ile Ala Arg Asp Asp
225                 230                 235                 240

Asn Asp Glu Glu Ile Met Gln Trp Leu Asp Lys Lys Glu Lys Gly Leu
                245                 250                 255

Thr Val Phe Val Ser Phe Gly Ser Glu Tyr Phe Leu Ser Lys Glu Asp
            260                 265                 270

Ile Phe Val Val Ala Arg Gly Leu Glu Leu Ser Lys Val Asn Phe Ile
        275                 280                 285

Trp Val Ile Arg Phe Ser Gln Gly Glu Arg Ile Ser Ile Gln Asp Ala
    290                 295                 300

Leu Pro Glu Gly Tyr Leu Glu Arg Val Gly Glu Arg Gly Met Val Ile
305                 310                 315                 320

Glu Gly Trp Ala Pro Gln Ala Met Ile Leu Gln His Pro Ser Ile Gly
                325                 330                 335

Gly Phe Val Ser His Cys Gly Trp Ser Ser Phe Met Glu Ser Met Lys
            340                 345                 350

Phe Gly Val Pro Ile Ile Ala Met Pro Met His Ile Asp Gln Pro Met
        355                 360                 365

Asn Ala Arg Leu Val Glu Tyr Ile Arg Met Gly Val Glu Ala Ala Arg
    370                 375                 380

Asp Glu Asn Gly Lys Leu Gln Ser Glu Glu Ile Ala Asn Thr Ile Arg
385                 390                 395                 400

Lys Val Leu Val Glu Glu Ser Gly Glu Asp Val Arg Asn Lys Ala Lys
                405                 410                 415

Glu Leu Ser Gly Lys Met Asn Ala Lys Gly Asp Glu Glu Ile Asp Gly
            420                 425                 430

Val Val Glu Glu Leu Met Ala Leu Cys Ser Asn Lys
        435                 440

<210> 3
<211> 454
<212> PRT
<213> Solanum lycopersicon

<400> 3

Met Asp Gly Ser Asn Glu Lys Ser Ile Arg Val Leu Met Phe Pro Trp
1               5                   10                  15

Leu Gly His Gly His Ile Ser Pro Phe Phe Glu Leu Ala Lys Lys Leu
            20                  25                  30

Val Lys Arg Asn Phe Thr Ile Phe Leu Val Ser Thr Pro Ala Asn Phe
        35                  40                  45

Ile Ser Ile Lys Gln Lys Leu Ile His Glu Asn Leu Cys Asp Lys Ile
    50                  55                  60

His Leu Phe Asp Leu Arg Leu Pro Ser Leu Pro Asp Leu Pro Pro His
65                  70                  75                  80

Tyr His Thr Thr Asn Gly Leu Pro Pro His Leu Met Ser Thr Leu Lys
                85                  90                  95

Lys Ala Phe Ala Lys Ser Arg Pro Ile Phe Thr Gln Ile Met Asn Thr
            100                 105                 110

Ile Glu Pro Asp Leu Leu Leu Tyr Asp Leu Leu Gln Pro Trp Ala Pro
        115                 120                 125

Lys Val Ala Lys Glu Lys Asn Ile Pro Ser Val Val Phe Val Thr Ser
    130                 135                 140

Ser Ala Thr Met Phe Ser Tyr Met Phe His Asn Phe Arg Tyr Pro Asn
145                 150                 155                 160

Ser Gln Phe Pro Phe Ser Ser Ile Tyr Tyr Arg Asp Tyr Glu Leu Thr
                165                 170                 175

Arg Leu Ile Lys Asn Gln Glu Met Glu Thr Ile Glu Gln His Gln Arg
            180                 185                 190

Asp Asn Lys Ser Val Lys Met Cys Phe Lys Arg Ser Thr Asn Ile Val
        195                 200                 205

Leu Ile Lys Gly Phe Lys Glu Ile Asp Gly Gln Tyr Cys Glu Tyr Ile
    210                 215                 220

Ser Ser Leu Thr Lys Lys Arg Val Val Pro Val Gly Pro Leu Val Gln
225                 230                 235                 240

Glu Gln Thr Ser Glu Asp Asn Asn Ser Gln Ile Leu Thr Trp Leu Asn
                245                 250                 255

Gln Lys Ser Lys Gly Ser Thr Ile Phe Val Ser Phe Gly Ser Glu Tyr
            260                 265                 270

Phe Leu Ser Gln Glu Asp Arg Glu Glu Ile Ala His Gly Leu Glu Gln
        275                 280                 285

Ser Arg Val Asn Phe Ile Trp Val Val Arg Phe Pro Lys Gly Glu Lys
    290                 295                 300

Leu Lys Leu Glu Gln Ala Leu Pro Arg Asp Phe Phe Lys Lys Val Gly
305                 310                 315                 320

Glu Arg Gly Met Val Val Glu Asp Trp Ala Pro Gln Ala Lys Ile Leu
                325                 330                 335

Gly Asn Pro Asn Ile Gly Gly Phe Val Ser His Cys Gly Trp Asn Ser
            340                 345                 350

Val Leu Glu Ser Met Lys Ile Gly Val Pro Ile Ile Ala Met Pro Met
        355                 360                 365

His Leu Asp Gln Pro Leu Asn Ala Arg Leu Val Glu Glu Val Gly Ile
    370                 375                 380

Gly Leu Glu Val Val Arg Asp Lys Asp Gly Lys Leu Asp Gly Glu Gln
385                 390                 395                 400

Ile Ser Glu Ile Ile Asn Lys Val Val Leu Glu Lys Glu Gly Glu Ser
                405                 410                 415

Ile Arg Glu Lys Ala Lys Lys Met Ser Glu Thr Ile Arg Val Lys Gly
            420                 425                 430

Asp Glu Glu Ile Asp Asp Val Val Gln Glu Leu Val Asn Leu Cys Lys
        435                 440                 445

Thr Arg Ser Asn Val Val
    450

<210>  4
<211>  446
<212>  PRT
<213>  Solanum lycopersicon

<400>  4

Met Ser Thr Thr Leu Lys Val Leu Met Phe Pro Phe Leu Ala Tyr Gly
1               5                   10                  15

His Ile Ser Pro Tyr Leu Asn Val Ala Lys Lys Leu Ala Asp Arg Gly
            20                  25                  30

Phe Leu Ile Tyr Leu Cys Ser Thr Pro Ile Asn Leu Lys Ser Thr Ile
        35                  40                  45

Asn Lys Ile Pro Glu Lys Tyr Ala Asp Ser Ile Gln Leu Ile Glu Leu
    50                  55                  60

His Leu Pro Glu Leu Pro Glu Leu Pro Pro His Tyr His Thr Thr Asn
65                  70                  75                  80

Gly Leu Pro Pro Asn Leu Asn His Ile Leu Arg Arg Ala Leu Lys Met
                85                  90                  95

Ser Lys Pro Asn Phe Ser Lys Ile Met Gln Asn Leu Lys Pro Asp Leu
            100                 105                 110

Leu Ile Tyr Asp Ile Leu Gln Gln Trp Ala Glu Asp Val Ala Thr Glu
        115                 120                 125

Leu Asn Ile Pro Ala Val Lys Leu Leu Thr Ser Gly Val Ala Val Phe
    130                 135                 140

Ser Tyr Phe Phe Asn Leu Thr Lys Lys Pro Glu Val Glu Phe Pro Tyr
145                 150                 155                 160

Pro Ala Ile Tyr Leu Arg Lys Ile Glu Leu Val Arg Trp Cys Glu Thr
                165                 170                 175

Leu Ser Lys His Asn Lys Glu Gly Glu Glu His Asp Asp Gly Leu Ala
            180                 185                 190

Tyr Gly Asn Met Gln Ile Met Leu Met Ser Thr Ser Lys Ile Leu Glu
        195                 200                 205

Ala Lys Tyr Ile Asp Tyr Cys Ile Glu Leu Thr Asn Trp Lys Val Val
    210                 215                 220

Pro Val Gly Ser Leu Val Gln Asp Ser Ile Thr Asn Asp Ala Ala Asp
225                 230                 235                 240

Asp Asp Met Glu Leu Ile Asp Trp Leu Gly Thr Lys Asp Glu Asn Ser
                245                 250                 255

Thr Val Phe Val Ser Phe Gly Ser Glu Tyr Phe Leu Ser Lys Glu Asp
            260                 265                 270

Val Glu Glu Val Ala Phe Gly Leu Glu Leu Ser Asn Val Asn Phe Ile
        275                 280                 285

Trp Val Val Arg Phe Pro Lys Gly Glu Glu Lys Asn Leu Glu Asp Val
    290                 295                 300

Leu Pro Lys Gly Phe Phe Glu Arg Ile Gly Glu Arg Gly Arg Val Leu
305                 310                 315                 320

Asp Lys Phe Ala Pro Gln Pro Arg Ile Leu Asn His Pro Ser Thr Gly
                325                 330                 335

Gly Phe Ile Ser His Cys Gly Trp Asn Ser Ala Met Glu Ser Ile Asp
            340                 345                 350

Phe Gly Val Pro Ile Val Ala Met Pro Met Gln Leu Asp Gln Pro Met
        355                 360                 365

Asn Ala Arg Leu Ile Val Glu Leu Gly Val Ala Val Glu Ile Val Arg
    370                 375                 380

Asp Asp Asp Gly Lys Ile Tyr Arg Gly Glu Ile Ala Glu Thr Leu Lys
385                 390                 395                 400

Gly Val Ile Thr Gly Glu Ile Gly Glu Ile Leu Arg Ala Lys Val Arg
                405                 410                 415

Asp Ile Ser Lys Asn Leu Lys Ala Ile Lys Asp Glu Glu Met Asp Val
            420                 425                 430

Ala Ala Gln Glu Leu Ile Gln Leu Cys Arg Asn Ser Asn Lys
        435                 440                 445

<210>  5
<211>  473

<212>  PRT
<213>  Stevia rebaudiana

<400>  5

Met Ala Thr Ser Asp Ser Ile Val Asp Asp Arg Lys Gln Leu His Val
1               5                   10                  15

Ala Thr Phe Pro Trp Leu Ala Phe Gly His Ile Leu Pro Tyr Leu Gln
            20                  25                  30

Leu Ser Lys Leu Ile Ala Glu Lys Gly His Lys Val Ser Phe Leu Ser
        35                  40                  45

Thr Thr Arg Asn Ile Gln Arg Leu Ser Ser His Ile Ser Pro Leu Ile
    50                  55                  60

Asn Val Val Gln Leu Thr Leu Pro Arg Val Gln Glu Leu Pro Glu Asp
65                  70                  75                  80

Ala Glu Ala Thr Thr Asp Val His Pro Glu Asp Ile Pro Tyr Leu Lys
                85                  90                  95

Lys Ala Ser Asp Gly Leu Gln Pro Glu Val Thr Arg Phe Leu Glu Gln
            100                 105                 110

His Ser Pro Asp Trp Ile Ile Tyr Asp Tyr Thr His Tyr Trp Leu Pro
        115                 120                 125

Ser Ile Ala Ala Ser Leu Gly Ile Ser Arg Ala His Phe Ser Val Thr
    130                 135                 140

Thr Pro Trp Ala Ile Ala Tyr Met Gly Pro Ser Ala Asp Ala Met Ile
145                 150                 155                 160

Asn Gly Ser Asp Gly Arg Thr Thr Val Glu Asp Leu Thr Thr Pro Pro
                165                 170                 175

Lys Trp Phe Pro Phe Pro Thr Lys Val Cys Trp Arg Lys His Asp Leu
            180                 185                 190

Ala Arg Leu Val Pro Tyr Lys Ala Pro Gly Ile Ser Asp Gly Tyr Arg
        195                 200                 205

Met Gly Leu Val Leu Lys Gly Ser Asp Cys Leu Leu Ser Lys Cys Tyr
    210                 215                 220

His Glu Phe Gly Thr Gln Trp Leu Pro Leu Leu Glu Thr Leu His Gln
225                 230                 235                 240

Val Pro Val Val Pro Val Gly Leu Leu Pro Pro Glu Ile Pro Gly Asp
                245                 250                 255

Glu Lys Asp Glu Thr Trp Val Ser Ile Lys Lys Trp Leu Asp Gly Lys
            260                 265                 270

Gln Lys Gly Ser Val Val Tyr Val Ala Leu Gly Ser Glu Val Leu Val
        275                 280                 285

Ser Gln Thr Glu Val Val Glu Leu Ala Leu Gly Leu Glu Leu Ser Gly
    290                 295                 300

Leu Pro Phe Val Trp Ala Tyr Arg Lys Pro Lys Gly Pro Ala Lys Ser
305                 310                 315                 320

Asp Ser Val Glu Leu Pro Asp Gly Phe Val Glu Arg Thr Arg Asp Arg
                325                 330                 335

Gly Leu Val Trp Thr Ser Trp Ala Pro Gln Leu Arg Ile Leu Ser His
            340                 345                 350

Glu Ser Val Cys Gly Phe Leu Thr His Cys Gly Ser Gly Ser Ile Val
        355                 360                 365

Glu Gly Leu Met Phe Gly His Pro Leu Ile Met Leu Pro Ile Phe Gly
    370                 375                 380

Asp Gln Pro Leu Asn Ala Arg Leu Leu Glu Asp Lys Gln Val Gly Ile
385                 390                 395                 400

Glu Ile Pro Arg Asn Glu Glu Asp Gly Cys Leu Thr Lys Glu Ser Val
                405                 410                 415

Ala Arg Ser Leu Arg Ser Val Val Val Glu Lys Glu Gly Glu Ile Tyr
            420                 425                 430

Lys Ala Asn Ala Arg Glu Leu Ser Lys Ile Tyr Asn Asp Thr Lys Val
        435                 440                 445

Glu Lys Glu Tyr Val Ser Gln Phe Val Asp Tyr Leu Glu Lys Asn Ala
    450                 455                 460

Arg Ala Val Ala Ile Asp His Glu Ser
465                 470

<210>  6

<211>  481
<212>  PRT
<213>  Stevia rebaudiana

<400>  6

Met Asp Ala Met Ala Thr Thr Glu Lys Lys Pro His Val Ile Phe Ile
1               5                   10                  15

Pro Phe Pro Ala Gln Ser His Ile Lys Ala Met Leu Lys Leu Ala Gln
            20                  25                  30

Leu Leu His His Lys Gly Leu Gln Ile Thr Phe Val Asn Thr Asp Phe
        35                  40                  45

Ile His Asn Gln Phe Leu Glu Ser Ser Gly Pro His Cys Leu Asp Gly
    50                  55                  60

Ala Pro Gly Phe Arg Phe Glu Thr Ile Pro Asp Gly Val Ser His Ser
65                  70                  75                  80

Pro Glu Ala Ser Ile Pro Ile Arg Glu Ser Leu Leu Arg Ser Ile Glu
                85                  90                  95

Thr Asn Phe Leu Asp Arg Phe Ile Asp Leu Val Thr Lys Leu Pro Asp
            100                 105                 110

Pro Pro Thr Cys Ile Ile Ser Asp Gly Phe Leu Ser Val Phe Thr Ile
        115                 120                 125

Asp Ala Ala Lys Lys Leu Gly Ile Pro Val Met Met Tyr Trp Thr Leu
    130                 135                 140

Ala Ala Cys Gly Phe Met Gly Phe Tyr His Ile His Ser Leu Ile Glu
145                 150                 155                 160

Lys Gly Phe Ala Pro Leu Lys Asp Ala Ser Tyr Leu Thr Asn Gly Tyr
                165                 170                 175

Leu Asp Thr Val Ile Asp Trp Val Pro Gly Met Glu Gly Ile Arg Leu
            180                 185                 190

Lys Asp Phe Pro Leu Asp Trp Ser Thr Asp Leu Asn Asp Lys Val Leu
        195                 200                 205

Met Phe Thr Thr Glu Ala Pro Gln Arg Ser His Lys Val Ser His His
    210                 215                 220

Ile Phe His Thr Phe Asp Glu Leu Glu Pro Ser Ile Ile Lys Thr Leu

225                 230                 235                 240

Ser Leu Arg Tyr Asn His Ile Tyr Thr Ile Gly Pro Leu Gln Leu Leu
                245                 250                 255

Leu Asp Gln Ile Pro Glu Glu Lys Lys Gln Thr Gly Ile Thr Ser Leu
            260                 265                 270

His Gly Tyr Ser Leu Val Lys Glu Glu Pro Glu Cys Phe Gln Trp Leu
        275                 280                 285

Gln Ser Lys Glu Pro Asn Ser Val Val Tyr Val Asn Phe Gly Ser Thr
    290                 295                 300

Thr Val Met Ser Leu Glu Asp Met Thr Glu Phe Gly Trp Gly Leu Ala
305                 310                 315                 320

Asn Ser Asn His Tyr Phe Leu Trp Ile Ile Arg Ser Asn Leu Val Ile
                325                 330                 335

Gly Glu Asn Ala Val Leu Pro Pro Glu Leu Glu Glu His Ile Lys Lys
            340                 345                 350

Arg Gly Phe Ile Ala Ser Trp Cys Ser Gln Glu Lys Val Leu Lys His
        355                 360                 365

Pro Ser Val Gly Gly Phe Leu Thr His Cys Gly Trp Gly Ser Thr Ile
    370                 375                 380

Glu Ser Leu Ser Ala Gly Val Pro Met Ile Cys Trp Pro Tyr Ser Trp
385                 390                 395                 400

Asp Gln Leu Thr Asn Cys Arg Tyr Ile Cys Lys Glu Trp Glu Val Gly
                405                 410                 415

Leu Glu Met Gly Thr Lys Val Lys Arg Asp Glu Val Lys Arg Leu Val
            420                 425                 430

Gln Glu Leu Met Gly Glu Gly Gly His Lys Met Arg Asn Lys Ala Lys
        435                 440                 445

Asp Trp Lys Glu Lys Ala Arg Ile Ala Ile Ala Pro Asn Gly Ser Ser
    450                 455                 460

Ser Leu Asn Ile Asp Lys Met Val Lys Glu Ile Thr Val Leu Ala Arg
465                 470                 475                 480

Asn

<210> 7
<211> 32
<212> DNA
<213> Artificial sequence

<220>
<223> RT7 forward primer

<400> 7
attaggatcc aatgggaaca caagtaacag ag 32

<210> 8
<211> 37
<212> DNA
<213> Artificial sequence

<220>
<223> RT7 reverse primer

<400> 8
aatactgcag ttaattagta ctaatcttac aaaattg 37

<210> 9
<211> 37
<212> DNA
<213> Artificial sequence

<220>
<223> RT11 forward primer

<400> 9
attaggatcc aatggaagcc aagaaaaata aaatgag 37

<210> 10
<211> 35
<212> DNA
<213> Artificial sequence

<220>
<223> RT11 reverse primer

<400> 10
aatactgcag tcatttgttg ctgcaaagag ccatc 35

<210> 11
<211> 34
<212> DNA
<213> Artificial sequence

<220>
<223> RT15 forward primer

<400> 11
attaggatcc aatggatggt tcgaatgaaa agtc 34

<210>  12
<211>  35
<212>  DNA
<213>  Artificial sequence

<220>
<223>  RT15 reverse primer

<400>  12
aatactgcag ctagacaaca tttgatctag tcttg                                35


<210>  13
<211>  38
<212>  DNA
<213>  Artificial sequence

<220>
<223>  RT18 forward primer

<400>  13
attaggatcc aatgagtact actttaaagg tattgatg                             38


<210>  14
<211>  36
<212>  DNA
<213>  Artificial sequence

<220>
<223>  RT reverse primer

<400>  14
aatactgcag attcacttat tactattcct acaaag                               36


<210>  15
<211>  31
<212>  DNA
<213>  Artificial sequence

<220>
<223>  UGT2_1a forward primer

<400>  15
attaggatcc aatggccact tctgactcca t                                    31


<210>  16
<211>  32
<212>  DNA
<213>  Artificial sequence

<220>
<223>  UGT2_1a reverse primer

<400>  16
aataaagctt ttagctttcg tggtcaatgg ca                                   32


<210>  17
<211>  32

<212>  DNA
<213>  Artificial sequence

<220>
<223>  UGT85C2 forward primer

<400>  17
attaggatcc aatggacgct atggccacca ct                                    32


<210>  18
<211>  32
<212>  DNA
<213>  Artificial sequence

<220>
<223>  UGT85C2 reverse primer

<400>  18
aataaagctt ttagtttcga gccaagacag tg                                    32


<210>  19
<211>  1341
<212>  DNA
<213>  Artificial sequence

<220>
<223>  RT18 open reading frame optimized for expression in Y. lipolitica

<400>  19
atgtccacca ccctcaaggt cctcatgttc cccttcctcg cttacggcca catctctccc      60

tacctcaacg ttgccaagaa gctcgccgac cgaggcttcc tcatctacct ctgttccacc     120

cccatcaacc tcaagtccac catcaacaag atccccgaga agtacgccga ctccatccag     180

ctcatcgaac tccatctccc cgagcttccc gagctgcctc cccactacca caccaccaac     240

ggtctgcctc ccaacctcaa ccacatcctc cgacgagccc tcaagatgtc caagcccaac     300

ttctccaaga tcatgcagaa cctgaagccc gatctgctca tctacgacat tctccagcag     360

tgggccgagg atgtcgccac cgagcttaac atccccgccg tcaagctgct cacctctggt     420

gttgctgttt tctcttactt cttcaacctc accaagaagc ccgaggtcga gttcccctac     480

cccgctatct acctccgaaa gatcgagctg gtccgatggt gcgagactct gtccaagcac     540

aacaaggaag gtgaggagca cgacgacggc ctcgcctacg gcaacatgca gatcatgctc     600

atgtccactt ccaagatcct cgaggccaag tacattgact actgcattga gctgaccaac     660

tggaaggtcg tccccgtcgg ctctctcgtc caggactcca tcaccaacga cgccgctgac     720

gacgacatgg aactcattga ctggctcggt actaaggacg agaactccac cgtctttgtc     780

tcttttggct ccgagtactt cctctccaaa gaggacgttg aagaggttgc cttcggtctg     840

gagctgtcca acgtcaactt catctgggtt gtccgattcc ccaagggtga ggagaagaac     900

ctcgaggacg ttctgcccaa gggcttcttc gagcgaatcg gtgagcgagg ccgagtcctc     960

gacaagtttg ctccccagcc ccgaattctc aaccacccct ctaccggtgg tttcatctct 1020

cactgtggct ggaactccgc catggagtcc attgactttg gtgtccccat tgtcgccatg 1080

cccatgcagc tcgaccagcc catgaacgcc cgactcattg tcgagcttgg tgttgccgtc 1140

gagattgtcc gagatgatga tggtaagatc taccgaggtg agattgctga gactctcaag 1200

ggtgtcatca ccggcgagat tggtgagatc ctccgagcca aggtccgaga catctccaag 1260

aacctcaagg ccatcaagga cgaggagatg gacgttgctg cccaggagct gatccagctc 1320

tgccgaaact ccaataaata a 1341

<210> 20
<211> 866
<212> DNA
<213> Yarrowia lipolytica

<400> 20
catgctcact tttgttgtcc tgatgatctc ccgttatttc gccgctcctc tggaaaccat 60

ccgcccgcaa atcccctctg cccatcttga caatgcacaa tgcatcattc tcagcctgca 120

tgaatgcgaa agatggcaat attggtggag gaggcgacgg cggtaaacaa tggagataga 180

aaccacaaaa gaaacctgga aacccaaaat ggactcacga caactccccc actcccccac 240

tccccatctc cccctgggca tcagttgccc atcggtatct caactgtcgc actagttagc 300

gcaaccatca catactttag acgccaaaca atgggacaac tcatcgcgcc gaactatggg 360

cagattttaa ctcgcacaac attaccccaa ctctaaaagg taacctcgac cggaaaacgg 420

gaagacagga tcagcaaccg tgatcgacag aatcttcagg gcactacagt tgatagacat 480

aggttatgtt ggtaggtcta gacgggcctc ggggaattga ccccaccagt tgcaagtcac 540

gtgcccctga tacagctagt ttagcacatc tgcccactac gtctggacgc accatggtgg 600

tgccagtcgc gtgaactcaa acacccacta gcctcgggaa ggattcagtt aaatccgcac 660

cttatttcca acacaaagaa gcggttggcg gacaaagaac atgtcctttc tggggcactg 720

tacattccag gactctgttc aaggtcaaat atacaaaaca cagatagaga aacatagaca 780

gctgcggcct tataaatacc tgggcgcact tctctctttt tccctcctca tcacacattc 840

gttcaccact aagtcactcg ttcaaa 866

<210> 21
<211> 999
<212> DNA
<213> Yarrowia lipolytica

<400> 21
ctactgttgc tacgattccc ccattgcaac cacagtttgg ggttaccccg cattatatta 60

gcatgattac gaaagagata agtatcatat ggaacatgtg aagggtagta tgcaggtccg 120

gcggagaaag agaatgacgt tttcattaag cgattcgctt ggcggcttgt gggggatgtg 180

acgatactta cggtaaagac cctgtgtgag agctggtact cgctcgttac ttcgctgatc 240

tgttgggccg tcaatcgaat ctcgtggaac ttgcattctt cttaactgtg tctatacaag 300

acacctaatg aaacatacaa gctaccgaaa tcattttact cgtactgacc ggtacggtac 360

ttgcacaagt agtgaaactt ccgaaaatag ccagcctcat gcatcatcgc ttcacccctt 420

ctgttgacct caaaagcatt ccaacggtaa aaaattataa cgccgccaac tggatggttg 480

tgacggcgtt gaccaccaat gtgtgggggc tggcggtagg accgagctta ttcgtcccaa 540

taagctcttt ggatttgatt ctttggggtg tgtggtaaaa ttcacatggg gaagaacacg 600

gtggcagttt gaggcagagg cccagcgtgt agttcctagg gcatgaatat accgaactca 660

tggcgcagaa ttgagctgaa tgcgcaaaaa gctacaggat caaccgcgtt agaaatgccg 720

caaatgtcca ctaattcccc ggactgttcc aaatgattct gtggggataa atctcaaact 780

gggttaggct ttgtcacgtt tctttgtgtc gtgtcggttc gtccggggca atgtgcccac 840

gcttggctgt ctccctacac ctcggtaaaa actatcacat gctgcccctc tcgagcaagc 900

attaaatgca tatagtcaat ctaacgacat atatataggt agggtgcatc ctccggttta 960

gctccccaga atatctctta ttcattacac aaaaacaac 999

<210> 22
<211> 1000
<212> DNA
<213> Yarrowia lipolytica

<400> 22
gtgcaatcac atgttgctac tgtacctgct gtggaccacg cacggcggaa cgtaccgtac 60

aaatattttc ttgctcacat gactctctct cggccgcgca cgccggtggc aaattgctct 120

tgcattggct ctgtctctag acgtccaaac cgtccaaagt ggcagggtga cgtgatgcga 180

cgcacgaagg agatggcccg gtggcgagga accggacacg gcgagccggc gggaaaaaag 240

gcggaaaacg aaaagcgaag ggcacaatct gacggtgcgg ctgccaccaa cccaaggagg 300

ctattttggg tcgctttcca tttcacattc gccctcaatg gccactttgc ggtggtgaac 360

atggtttctg aaacaacccc ccagaattag agtatattga tgtgtttaag attgggttgc 420

tatttggcca ttgtggggga gggtagcgac gtggaggaca ttccagggcg aattgagcct 480

agaaagtggt agcattccaa ccgtctaagt cgtccgaatt gatcgctata actatcacct 540

ctctcacatg tctacttccc caaccaacat ccccaacctc ccccacacta aagttcacgc 600

caataatgta ggcactcttt ctgggtgtgg gacagcagag caatacggag gggagattac 660

acaacgagcc acaattgggg agatggtagc catctcactc gacccgtcga cttttggcaa 720

cgctcaatta cccaccaaat ttgggctgga gttgagggga ccgtgttcca gcgctgtagg 780

accagcaaca cacacggtat caacagcaac caacgccccc gctaatgcac ccagtactgc 840

gcaggtgtgg gccaggtgcg ttccagatgc gagttggcga accctaagcc gacagtgtac 900

tttttgggac gggcagtagc aatcgtgggc ggaaaccccg gtgtatataa aggggtggag 960

aggacggatt attagcacca acacacacac ttatactaca 1000

<210> 23
<211> 1503
<212> DNA
<213> Artificial sequence

<220>
<223> tHMG optimized for expression in Y. lipoltica

<400> 23
atgacccagt ctgtgaaggt ggttgagaag cacgttccta tcgtcattga gaagcccagc 60

gagaaggagg aggacacctc ttctgaagac tccattgagc tgactgtcgg aaagcagccc 120

aagcccgtga ccgagacccg ttctctggac gacttggagg ctatcatgaa ggcaggtaag 180

accaagctcc tggaggacca cgaggttgtc aagctctctc tcgaaggcaa gctccctttg 240

tatgctcttg agaagcagct tggtgacaac acccgagctg ttggcatccg acgatctatc 300

atctcccagc agtctaatac caagactctt gagacctcaa agctccctta cctgcactac 360

gactacgacc gtgtttttgg agcctgttgc gagaacgtta ttggttacat gcctctcccc 420

gttggtgttg ctggccccat gaacattgat ggcaagaact accacattcc tatggccacc 480

actgagggtt gtcttgttgc ctcaaccatg cgaggttgca aggccatcaa cgccggtggc 540

ggtgttacca ctgtgcttac tcaggacggt atgacacgag gtccttgtgt ttccttcccc 600

tctctcaagc gggctggagc cgctaagatc tggcttgatt ccgaggaggg tctcaagtcc 660

atgcgaaagg ccttcaactc cacctctcga tttgctcgtc tccagtctct tcactctacc 720

cttgctggta acctgctgtt tattcgattc cgaaccacca ctggtgatgc catgggcatg 780

aacatgatct ccaagggcgt cgaacactct ctggccgtca tggtcaagga gtacggcttc 840

cctgatatgg acattgtgtc tgtctcgggt aactactgca ctgacaagaa gcccgcagcg 900

atcaactgga tcgaaggccg aggcaagagt gttgttgccg aagccaccat ccctgctcac 960

attgtcaagt ctgttctcaa aagtgaggtt gacgctcttg ttgagctcaa catcagcaag 1020

aatctgatcg gtagtgccat ggctggctct gtgggaggtt tcaatgcaca cgccgcaaac 1080

ctggtgaccg ccatctacct tgccactggc caggatcctg ctcagaatgt cgagtcttcc 1140

aactgcatca cgctgatgag caacgtcgac ggtaacctgc tcatctccgt ttccatgcct 1200

tctatcgagg tcggtaccat tggtggaggt actattttgg agccccaggg tgctatgctg 1260

gagatgcttg gcgtgcgagg tcctcacatc gagacccccg gtgccaacgc ccaacagctt 1320

gctcgcatca ttgcttctgg agttcttgca gcggagcttt cgctgtgttc tgctcttgct 1380

gccggccatc ttgtgcaaag tcatatgacc cacaaccgtt cccaggctcc tactccggcc 1440

aagcagtctc aggccgatct gcagcgtctc caaaacggtt cgaatatctg cattcggtca 1500

tag 1503

<210> 24
<211> 984
<212> DNA
<213> Artificial sequence

<220>
<223> GGS optimized for expression in Y. lipolitica

<400> 24
atggattata acagcgcgga tttcaaggag atctggggca aggccgccga caccgcgctg 60

ctgggaccgt acaactacct cgccaacaac cggggccaca acatcagaga acacttgatc 120

gcagcgttcg gagcggttat caaggtggac aagagcgatc tcgaaaccat ttcgcacatc 180

accaagattt tgcataactc gtcgctgctt gttgatgacg tggaagacaa ctcgatgctc 240

cgacgaggcc tgccggcagc ccattgtctg tttggagtcc cccaaaccat caactccgcc 300

aactacatgt actttgtggc tctgcaggag gtgctcaagc tcaagtctta tgatgccgtc 360

tccattttca ccgaggaaat gatcaacttg catagaggtc agggtatgga tctctactgg 420

agagaaacac tcacttgccc ctcggaagac gagtatctgg agatggtggt gcacaagacc 480

ggaggactgt ttcggctggc tctgagactt atgctgtcgg tggcatcgaa acaggaggac 540

catgaaaaga tcaactttga tctcacacac cttaccgaca cactgggagt catttaccag 600

attctggatg attacctcaa cctgcagtcc acggaattga ccgagaacaa gggattctgc 660

gaagatatca gcgaaggaaa gttttcgttt ccgctgattc acagcatccg gaccaacccg 720

gataaccacg agattctcaa cattctcaaa cagcgaacaa gcgacgcttc actcaaaaag 780

tacgccgtgg actacatgag aacagaaacc aagagtttcg actactgcct caagagaatc 840

caggccatgt cactcaaggc aagttcgtac attgatgatc tcgcagcagc cggccacgat 900

gtctccaagt tgcgagccat tttgcattat tttgtgtcca cctctgactg tgaggagaga 960

aagtactttg aggatgcgca gtga 984

<210> 25
<211> 2232
<212> DNA
<213> Artificial sequence

<220>
<223> CPS from S. rebaudiana optimized for expression in Y. lipolitica

<400> 25
atgtgcaagg ctgtttccaa ggagtactcc gatctgctcc agaaggacga ggcctctttc 60

accaagtggg acgacgacaa ggtcaaggac cacctcgaca ccaacaagaa cctctacccc 120

aacgacgaga tcaaggagtt tgtcgagtcc gtcaaggcca tgttcggctc catgaacgac 180

ggcgagatta atgtctctgc ttacgacacc gcctgggttg ctctggtcca ggatgtcgac 240

ggttccggct ctcctcagtt cccttcctct ctcgagtgga tcgccaacaa ccagctgtcc 300

gacggttctt ggggtgacca cctgctcttc tctgctcacg accgaatcat caacaccctg 360

gcctgtgtca ttgctctgac ctcttggaac gtccacccct ccaagtgcga gaagggtctg 420

aacttcctcc gagagaacat ctgcaagctc gaggacgaga acgccgagca catgcccatt 480

ggcttcgagg tcaccttccc ctctctgatt gacattgcca agaagctcaa cattgaggtc 540

cccgaggaca cccccgctct caaggagatc tacgctcgac gagacatcaa gctcaccaag 600

atccccatgg aggttctcca caaggtcccc accactctcc tccactctct cgagggtatg 660

cccgatctcg agtgggagaa gctgctcaag ctgcagtgca aggacggctc tttcctcttc 720

tccccctctt ccactgcctt cgccctcatg cagaccaagg acgagaagtg tctccagtac 780

ctcaccaaca ttgtcaccaa gttcaacggt ggtgtcccca acgtctaccc cgttgacctc 840

tttgagcaca tctgggttgt tgaccgactc cagcgactcg gtatcgcccg atacttcaag 900

tccgagatca aggactgtgt cgagtacatc aacaagtact ggaccaagaa cggtatctgc 960

tgggcccgaa acacccacgt ccaggacatt gacgacaccg ccatgggctt ccgagttctg 1020

cgagcccacg gctacgatgt cacccccgat gtctttcgac agtttgagaa ggacggcaag 1080

tttgtctgtt tcgccggtca gtccacccag gccgtcaccg gtatgttcaa cgtctaccga 1140

gcttctcaga tgctcttccc cggtgagcga atcctcgagg acgccaagaa gttctcctac 1200

aactacctca aggagaagca gtccaccaac gagctgctcg acaagtggat cattgccaag 1260

gatctgcccg gtgaggttgg ctacgccctc gacatcccct ggtacgcctc tctgccccga 1320

ctggagactc gatactacct cgagcagtac ggtggtgagg acgatgtctg gatcggtaag 1380

accctgtacc gaatgggcta cgtttccaac aacacctacc tcgagatggc caagctcgac 1440

tacaacaact acgttgccgt cctccagctc gagtggtaca ccatccagca gtggtacgtc 1500

gacattggta tcgagaagtt cgagtccgac aacatcaagt ccgtccttgt ctcctactac 1560

ctcgctgctg cctccatctt cgagcccgag cgatccaagg agcgaattgc ctgggccaag 1620

accaccatcc tcgtcgacaa gatcacctcc atcttcgact cctcccagtc ctccaaggaa 1680

gatatcaccg ccttcattga caagttccga aacaagtcct cctccaagaa gcactccatc 1740

aacggcgagc cctggcacga ggtcatggtt gctctcaaga aaactctcca cggctttgcc 1800

ctcgacgctc tgatgaccca ctctcaggac atccaccccc agctccacca ggcctgggag 1860

atgtggctca ccaagctcca ggacggtgtt gatgtcactg ctgagctcat ggtccagatg 1920

atcaacatga ccgccggccg atgggtttcc aaggagctcc tcacccaccc ccagtaccag 1980

cgactctcca ctgtcaccaa ctctgtctgc cacgacatca ccaagctcca caacttcaag 2040

gagaactcca ccaccgtcga ctccaaggtc caggagctgg tccagctcgt tttctccgac 2100

acccccgatg atctcgacca ggacatgaag cagaccttcc tgactgtcat gaaaactttc 2160

tactacaagg cctggtgcga ccccaacacc atcaacgacc acatctccaa ggtctttgag 2220

attgtgattt aa 2232

<210> 26
<211> 2274
<212> DNA
<213> Artificial sequence

<220>
<223> tKS from S. rebaudiana optimized for expression in Y. lipoltica

<400> 26
atgacctccc acggcggcca gaccaacccc accaacctca tcattgacac caccaaggag 60

cgaatccaga agcagttcaa gaacgtcgag atctccgttt cctcctacga caccgcctgg 120

gtcgccatgg tcccctctcc caactccccc aagtctccct gcttccccga gtgtctcaac 180

tggctcatca acaaccagct caacgacggc tcttggggtc tggtcaacca cacccacaac 240

cacaaccacc ccctcctcaa ggactctctc tcttccactc tcgcctgcat tgttgctctc 300

aagcgatgga acgttggcga ggaccagatc aacaagggtc tgtctttcat tgagtccaac 360

ctcgcctccg ccaccgagaa gtcccagccc tcccccattg gctttgatat catcttcccc 420

ggtctgctcg agtacgccaa gaacctcgat atcaacctgc tctccaagca gaccgacttc 480

tctctcatgc tgcacaagcg agagctcgag cagaagcgat gccactccaa cgagatggac 540

ggctacctgg cctacatttc cgagggtctg ggtaacctct acgactggaa catggtcaag 600

aagtaccaga tgaagaacgg ttccgttttc aactccccct ctgccaccgc tgctgccttc 660

atcaaccacc agaaccccgg ctgtctcaac tacctcaact ctctgctcga caagtttggt 720

aacgccgtcc ccactgtcta cccccacgat ctcttcatcc gactctccat ggtcgacacc 780

attgagcgac tcggtatttc ccaccacttc cgagtcgaga tcaagaacgt tctcgatgag 840

acttaccgat gctgggttga gcgagatgag cagatcttca tggacgttgt cacctgtgct 900

ctggccttcc gactcctccg aatcaacggt tacgaggttt cccccgaccc cctcgccgag 960

atcaccaacg agctggctct caaggacgag tacgccgccc tcgagactta ccacgcttct 1020

cacattctgt accaagagga tctgtcctcc ggcaagcaga ttctcaagtc cgccgacttc 1080

ctcaaggaga tcatctccac tgactccaac cgactctcca agctcatcca caaggaagtc 1140

gagaacgctc tcaagttccc catcaacacc ggtctggagc gaatcaacac ccgacgaaac 1200

atccagctct acaacgtcga caacacccga attctcaaga ccacctacca ctcttccaac 1260

atctccaaca ccgactacct gcgactcgcc gtcgaggact tctacacctg ccagtccatc 1320

taccgagagg agctcaaggg tctggagcga tgggttgtcg agaacaagct cgaccagctc 1380

aagtttgccc gacaaaagac tgcctactgc tacttctccg ttgctgccac cctctcttct 1440

cccgagctct ccgacgcccg aatctcttgg gccaagaacg gtatcctgac cactgttgtc 1500

gacgacttct ttgacattgg tggcaccatt gacgagctga ccaacctcat ccagtgcgtc 1560

gagaagtgga acgtcgacgt tgacaaggac tgttgttccg agcacgtccg aatcctcttc 1620

ctggctctca aggacgccat ctgctggatc ggtgacgagg ccttcaagtg gcaggctcga 1680

gatgtcactt cccacgtcat ccagacctgg ctcgagctca tgaactccat gctgcgagag 1740

gccatctgga cccgagatgc ctacgtcccc accctcaacg agtacatgga gaacgcctac 1800

gtcagctttg ctctcggtcc cattgtcaag cccgccatct actttgtcgg tcccaagctg 1860

tccgaggaga ttgtcgagtc ctccgagtac cacaacctct tcaagctcat gtccacccag 1920

ggccgactcc tcaacgatat ccactccttc aagcgagagt tcaaggaagg taagctcaac 1980

gccgttgctc tgcacctgtc caacggtgag tccggcaagg tcgaggaaga ggtcgtcgag 2040

gagatgatga tgatgatcaa gaacaagcga aaggagctca tgaagctcat cttcgaggag 2100

aacggctcca ttgtcccccg agcctgcaag gacgccttct ggaacatgtg ccacgtcctc 2160

aacttcttct acgccaacga cgacggtttc accggcaaca ccattctcga caccgtcaag 2220

gacatcatct acaaccctct ggttctggtc aacgagaacg aggagcagag gtaa 2274

<210> 27
<211> 1578
<212> DNA
<213> Artificial sequence

<220>
<223> KO from Gibberella fujikori optimized for expression in Y.
 lipolitica

<400> 27
atgtccaagt ccaactccat gaactccacc tcccacgaga ctctcttcca gcagctcgtt 60

ctcggcctcg accgaatgcc cctcatggac gtccactggc tcatctacgt tgcctttggt 120

gcctggctct gctcctacgt catccacgtt ctgtcctctt cctccactgt caaggtcccc 180

gtcgtcggtt accgatccgt tttcgagccc acctggctcc tccgactgcg attcgtctgg 240

gagggtggtt ccatcattgg ccagggctac aacaagttca aggactccat cttccaggtc 300

cgaaagctcg gtaccgacat tgtcatcatc cctcccaact acattgacga ggtccgaaag 360

ctctcccagg acaagacccg atccgtcgag cccttcatca acgactttgc cggccagtac 420

acccgaggta tggtctttct gcagtccgat ctccagaacc gagtcatcca gcagcgactc 480

acccccaagc ttgtctctct caccaaggtc atgaaggaag agctcgacta cgctctgacc 540

aaggagatgc ccgacatgaa gaacgacgag tgggttgagg tcgacatctc ttccatcatg 600

gtccgactca tctctcgaat ctccgcccga gttttcctcg gccccgagca ctgccgaaac 660

caggagtggc tcaccaccac cgccgagtac tccgagtctc tcttcatcac cggcttcatc 720

ctccgagttg tcccccacat tctccgaccc ttcattgctc ctctgctgcc ctcttaccga 780

accctgctgc gaaacgtttc ttccggccga cgagtcattg gtgatatcat ccgatcccag 840

cagggtgacg gtaacgagga catcctctct tggatgcgag atgctgccac tggtgaggag 900

aagcagatcg acaacattgc ccagcgaatg ctcattctgt ctctcgcctc catccacacc 960

accgccatga ccatgaccca cgccatgtac gatctgtgtg cctgccccga gtacattgag 1020

cccctccgag atgaggtcaa gtccgtcgtt ggtgcttctg gctgggacaa gaccgctctc 1080

aaccgattcc acaagctcga ctctttcctc aaggagtccc agcgattcaa ccccgttttc 1140

ctgctcacct tcaaccgaat ctaccaccag tccatgaccc tctccgatgg taccaacatc 1200

ccctccggta cccgaattgc tgtcccctct cacgccatgc tccaggactc cgcccacgtc 1260

cccggtccca ctcctcccac tgagttcgac ggtttccgat actccaagat ccgatccgac 1320

tccaactacg cccagaagta cctcttctcc atgaccgact cttccaacat ggcctttggc 1380

tacggtaagt acgcctgccc cggccgattc tacgcctcca acgagatgaa gctgactctg 1440

gccattctgc tcctccagtt tgagttcaag ctccccgacg gtaagggccg accccgaaac 1500

atcaccatcg actccgacat gatccccgac ccccgagctc gactctgtgt ccgaaagcga 1560

tctctgcgtg acgagtaa 1578

<210> 28
<211> 1578
<212> DNA
<213> Artificial sequence

<220>
<223> KAH_4 optimized for expression in Y. lipolitica

<400> 28
atggagtctc tggttgtcca caccgtcaac gccatctggt gcattgtcat tgtcggtatc 60

ttctccgtcg gctaccacgt ctacggccga gctgttgtcg agcagtggcg aatgcgacga 120

tctctcaagc tccagggtgt caagggtcct cctccctcca tcttcaacgg taacgtttcc 180

gagatgcagc gaatccagtc cgaggccaag cactgctccg gtgacaacat catctcccac 240

gactactctt cttctctgtt cccccacttt gaccactggc gaaagcagta cggccgaatc 300

tacacctact ccactggcct caagcagcac ctctacatca accaccccga gatggtcaag 360

gagctctccc agaccaacac cctcaacctc ggccgaatca cccacatcac caagcgactc 420

aaccccattc tcggtaacgg tatcatcacc tccaacggcc cccactgggc ccaccagcga 480

cgaatcattg cctacgagtt cacccacgac aagatcaagg gtatggtcgg tctgatggtc 540

gagtccgcca tgcccatgct caacaagtgg gaggagatgg tcaagcgagg tggtgagatg 600

ggctgtgaca tccgagtcga cgaggacctc aaggatgtct ccgctgacgt cattgccaag 660

gcctgtttcg gctcttcctt ctccaagggc aaggccatct tctccatgat ccgagatctg 720

ctcaccgcca tcaccaagcg atccgtcctc ttccgattca acggtttcac cgacatggtt 780

ttcggctcca agaagcacgg tgacgttgac attgacgctc tcgagatgga gctcgagtcc 840

tccatctggg agactgtcaa ggagcgagag attgagtgca aggacaccca caagaaggac 900

ctcatgcagc tcattctcga gggtgccatg cgatcttgtg acggtaacct gtgggacaag 960

tctgcttacc gacgattcgt tgtcgacaac tgcaagtcca tctactttgc cggccacgac 1020

tccaccgccg tttccgtttc ttggtgcctc atgctgctcg ctctcaaccc ctcttggcag 1080

gtcaagatcc gagatgagat tctgtcctcc tgcaagaacg gtatccccga cgccgagtcc 1140

atccccaacc tcaagaccgt caccatggtc atccaggaga ctatgcgact ctaccctccc 1200

gctcccattg tcggccgaga ggcctccaag gacattcgac tcggtgatct ggttgtcccc 1260

aagggtgtct gtatctggac cctcatcccc gctctgcacc gagatcccga gatctggggt 1320

cccgacgcca acgacttcaa gcccgagcga ttctccgagg gtatctccaa ggcctgcaag 1380

tacccccagt cctacatccc ctttggcctc ggcccccgaa cctgtgtcgg caagaacttt 1440

ggtatgatgg aggtcaaggt cctcgtttct ctgattgtct ccaagttctc cttcactctg 1500

tctcccacct accagcactc tccctcccac aagctgctcg tcgagcccca gcacggtgtt 1560

gtcatccgag ttgtataa 1578

<210> 29
<211> 2133
<212> DNA
<213> Artificial sequence

<220>
<223> CPR_optimized for expression in Y. lipolitica

<400> 29
atgtcctcct cttcttcttc ttccacctcc atgattgatc tcatggctgc catcatcaag 60

ggtgagcccg tcattgtctc cgaccccgcc aacgcctccg cctacgagtc cgttgctgcc 120

gagctgtcct ccatgctcat cgagaaccga cagtttgcca tgatcgtcac cacctccatt 180

gctgttctca ttggctgcat tgtcatgctc gtctggcgac gatctggctc cggtaactcc 240

aagcgagtcg agcccctcaa gcccctggtc atcaagcccc gagaagagga gatcgacgac 300

ggccgaaaga aggtcaccat cttctttggc acccagaccg gtactgctga gggcttcgcc 360

aaggctctcg gtgaggaagc caaggctcga tacgaaaaga cccgattcaa gattgtcgac 420

ctcgatgatt acgctgccga tgacgacgag tacgaggaga agctcaagaa agaggacgtt 480

gccttcttct tcctcgccac ctacggtgac ggtgagccca ccgacaacgc tgcccgattc 540

tacaagtggt tcaccgaggg taacgaccga ggcgagtggc tcaagaacct caagtacggt 600

gttttcggtc tgggcaaccg acagtacgag cacttcaaca aggttgccaa ggttgtcgac 660

gacatcctcg tcgagcaggg tgcccagcga ctcgtccagg tcggcctcgg tgatgatgac 720

cagtgcatcg aggacgactt cactgcctgg cgagaggctc tgtggcccga gctcgacacc 780

attctgcgag aggaaggtga caccgccgtt gccaccccct acaccgccgc cgtcctcgag 840

taccgagtct ccatccacga ctccgaggat gccaagttca acgacatcaa catggccaac 900

ggtaacggct acaccgtctt tgacgcccag cacccctaca aggccaacgt cgccgtcaag 960

cgagagctcc acacccccga gtccgaccga tcttgtatcc acctcgagtt tgacattgct 1020

ggttccggtc tgacctacga gactggtgac cacgttggtg tcctctgtga caacctgtcc 1080

gagactgtcg acgaggctct gcgactcctc gacatgtccc ccgacactta cttctctctg 1140

cacgccgaga aagaggacgg tactcccatc tcttcttctc tgccccctcc cttccctccc 1200

tgcaacctgc gaaccgctct gacccgatac gcctgcctcc tctcttctcc caagaagtct 1260

gctctcgttg ctctggccgc ccacgcctcc gaccccaccg aggctgagcg actcaagcac 1320

ctcgcctctc ccgctggcaa ggacgagtac tccaagtggg ttgtcgagtc ccagcgatct 1380

ctgctcgagg tcatggccga gttcccctcc gccaagcccc ctctcggtgt tttcttcgcc 1440

ggtgttgctc cccgactcca gccccgattc tactccatct cctcttcccc caagatcgcc 1500

gagactcgaa tccacgttac ctgtgctctg gtctacgaga agatgcccac cggccgaatc 1560

cacaagggtg tctgctccac ctggatgaag aacgccgttc cctacgagaa gtccgagaac 1620

tgttcctctg ctcccatctt tgtccgacag tccaacttca agctcccctc cgactccaag 1680

gtccccatca tcatgattgg ccccggtacc ggcctcgccc ccttccgagg cttcctgcag 1740

gagcgactcg ccctcgtcga gtccggtgtc gagctcggcc cctccgtcct cttctttggc 1800

tgccgaaacc gacgaatgga cttcatctac gaagaggagc tccagcgatt cgtcgagtcc 1860

ggtgctctcg ccgagctctc cgttgccttc tcccgagagg gtcccaccaa ggagtacgtc 1920

cagcacaaga tgatggacaa ggcctccgac atctggaaca tgatctccca gggcgcctac 1980

ctctacgtct gcggtgacgc caagggtatg gcccgagatg tccaccgatc tctgcacacc 2040

attgcccagg agcagggctc catggactcc accaaggccg agggtttcgt caagaacctc 2100

cagacctccg gccgatacct ccgagatgtc tgg 2133

<210> 30
<211> 1446
<212> DNA
<213> Artificial sequence

<220>
<223> UGT1 optimized for expression in Y. lipolitica

<400>  30
atggacgcca tggccaccac cgagaagaag ccccacgtca tcttcatccc cttccccgcc      60

cagtcccaca tcaaggccat gctcaagctc gcccagctcc tccaccacaa gggcctccag     120

atcacctttg tcaacaccga cttcatccac aaccagttcc tcgagtcctc cggcccccac     180

tgtctggacg gtgctcccgg tttccgattt gagactatcc ccgatggtgt ctcccactcc     240

cccgaggcct ccatccccat ccgagagtct ctgctccgat ccattgagac taacttcctc     300

gaccgattca ttgatctcgt caccaagctc cccgatcctc ccacctgtat catctccgac     360

ggtttcctgt ccgttttcac cattgatgct gccaagaagc tcggtatccc cgtcatgatg     420

tactggactc tggctgcctg tggtttcatg ggtttctacc acatccactc tctgatcgag     480

aagggctttg ctcctctcaa ggacgcctcc tacctcacca acggttacct cgacaccgtc     540

attgactggg tccccggtat ggagggtatc cgactcaagg acttccccct cgactggtcc     600

accgacctca acgacaaggt tctcatgttc accaccgagg ctccccagcg atcccacaag     660

gtttcccacc acatcttcca caccttcgac gagctcgagc cctccatcat caagactctg     720

tctctgcgat acaaccacat ctacaccatt ggccccctcc agctcctcct cgaccagatc     780

cccgaggaga agaagcagac cggtatcacc tctctgcacg gctactctct cgtcaaggaa     840

gagcccgagt gcttccagtg gctccagtcc aaggagccca actccgttgt ctacgtcaac     900

tttggctcca ccaccgtcat gtctctcgag gacatgaccg agtttggctg gggtctggcc     960

aactccaacc actacttcct gtggatcatc cgatccaacc tcgtcattgg cgagaacgcc    1020

gttctgcctc ccgagctcga ggagcacatc aagaagcgag gcttcattgc ctcttggtgc    1080

tcccaggaga aggttctcaa gcacccctcc gtcggtggtt tcctgaccca ctgcggctgg    1140

ggctccacca ttgagtctct gtccgctggt gtccccatga tctgctggcc ctactcctgg    1200

gaccagctca ccaactgccg atacatctgc aaggagtggg aggttggtct ggagatgggt    1260

accaaggtca agcgagatga ggtcaagcga ctcgtccagg agctcatggg cgagggtggt    1320

cacaagatgc gaaacaaggc caaggactgg aaggagaagg cccgaattgc cattgccccc    1380

aacggctctt cttctctcaa cattgacaag atggtcaagg agatcactgt tctcgctcga    1440

aactaa                                                               1446


<210>  31
<211>  1419
<212>  DNA
<213>  Artificial sequence

<220>
<223>  UGT2 variant optimized for expression in Y. lipolitica

<400>  31
atggccacct ccgactccat tgttgacgac cgaaagaagc tccacattgt catgttcccc      60

tggctcgcct ttggccacat catcccctat ctcgagcttt ccaagctcat tgcccagaag 120

ggccacaagg tttccttcct ctccaccacc aagaacattg accgactctc ctcccacatc 180

tctcccctca tcaactttgt caagctcacc ctcccccgag tccaggagct gcccgaggac 240

gccgaggcca ccactgatgt ccaccccgag gatatcccct acctcaagaa ggcctccgac 300

ggcctccagc ccgaggtcac tgagttcctc gagcagcact ctcccgactg gatcatctac 360

gactacaccc actactggct ccccgagatt gccaagtctc tcggtgtctc tcgagcccac 420

ttctccgtca ccaccccctg ggccattgct tacatgggtc ccactgccga tgccatgatc 480

aacggttccg actaccgaac cgagcttgag gacttcaccg tccctcccaa gtggttcccc 540

ttccccacca ccgtctgctg gcgaaagcac gatctggccc gactcgtccc ctacaaggct 600

cccggtatct ccgacggtta ccgaatgggc ctcgtcatca agggctgcga ctgtctgctc 660

tccaagacct accacgagtt cggtactcag tggctccgac ttctcgagga gctgcaccga 720

gtccccgtca tccccgttgg tctgctccct ccctccatcc ccggctctga caaggacgac 780

tcttgggttt ccatcaagga gtggctcgac ggccaggaga agggctccgt tgtctacgtt 840

gctctcggtt ccgaggttct cgtcacccag gaagaggttg tcgagcttgc tcacggtctg 900

gagctgtccg gtctgccctt cttctgggcc taccgaaagc ccaagggtcc cgccaagtcc 960

gactccgtcg agcttcccga tggtttcgtc gagcgagtcc gagatcgagg tctggtctgg 1020

acctcttggg ctccccagct ccgaatcctc tcccacgagt ccgttgctgg tttcctcacc 1080

cactgcggtt ccggctccat tgtcgagggc ctcatgttcg gccaccctct catcatgctc 1140

cccatcttcg gtgaccagcc cctcaacgcc cgactccttg aggacaagca ggtcggtatc 1200

gagatccccc gaaacgagga agatggttct ttcacccgag actctgttgc cgagtctctg 1260

cgactcgtca tggtcgagga agagggtaag atctaccgag agaaggccaa ggagatgtcc 1320

aagctctttg gcgacaagga cctccaggac cagtacgtcg acgactttgt cgagtacctc 1380

cagaagcacc gacgagctgt tgccattgac cacgaaagc 1419

<210> 32
<211> 1383
<212> DNA
<213> Artificial sequence

<220>
<223> UGT3 optimized for expression in Y. lipolitica

<400> 32
atggccgagc agcagaagat caagaagtct ccccacgttc tgctcatccc cttccctctg 60

cagggccaca tcaacccctt catccagttc ggcaagcgac tcatctccaa gggtgtcaag 120

accactctgg tcaccaccat ccacaccctc aactccactc tcaaccactc caacaccacc 180

accacctcca tcgagatcca ggccatctcc gacggctgtg acgagggtgg tttcatgtct 240

gctggtgagt cttacctcga gactttcaag caggtcggtt ccaagtctct ggctgacctc 300

atcaagaagc tccagtccga gggtaccacc attgacgcca tcatctacga ctccatgacc 360

gagtgggttc tcgatgtcgc catcgagttt ggtattgacg gtggctcctt cttcacccag 420

gcctgtgtcg tcaactctct ctactaccac gtccacaagg gtctgatctc tctgcccctc 480

ggcgagactg tctccgtccc cggtttcccc gttctgcagc gatgggagac tcctctcatt 540

ctccagaacc acgagcagat ccagtccccc tggtcccaga tgctcttcgg ccagttcgcc 600

aacattgacc aggcccgatg ggttttcacc aactccttct acaagctcga ggaagaggtc 660

attgagtgga cccgaaagat ctggaacctc aaggtcattg gccccaccct cccctccatg 720

tacctcgaca agcgactcga tgacgacaag gacaacggtt tcaacctcta caaggccaac 780

caccacgagt gcatgaactg gctcgacgac aagcccaagg agtccgttgt ctacgttgcc 840

tttggctctc tggtcaagca cggccccgag caggttgagg agatcacccg agctctgatt 900

gactccgatg tcaacttcct gtgggtcatc aagcacaagg aagagggtaa gctccccgag 960

aacctgtccg aggtcatcaa gaccggcaag ggcctcattg ttgcctggtg caagcagctc 1020

gacgttctcg cccacgagtc cgtcggctgc tttgtcaccc actgcggttt caactccacc 1080

ctcgaggcta tctctctcgg tgtccccgtt gttgccatgc cccagttctc cgaccagacc 1140

accaacgcca agctcctcga tgagattctc ggtgtcggtg tccgagtcaa ggctgacgag 1200

aacggtattg tccgacgagg taacctggct tcttgtatca agatgatcat ggaggaagag 1260

cgaggtgtca tcatccgaaa gaacgccgtc aagtggaagg atctggccaa ggttgctgtc 1320

cacgagggtg gctcttccga caacgacatt gtcgagtttg tctccgagct catcaaggcc 1380

taa 1383

<210> 33
<211> 1377
<212> DNA
<213> Artificial sequence

<220>
<223> UGT4 optimized for expression in Y. lipolitica

<400> 33
atggagaaca agaccgagac taccgtccga cgacgacgac gaatcattct cttccccgtc 60

cccttccagg gccacatcaa ccccattctg cagctcgcca acgttctgta ctccaagggc 120

ttctccatca ccatcttcca caccaacttc aacaagccca agacctccaa ctacccccac 180

ttcactttcc gattcatcct cgacaacgac ccccaggacg agcgaatctc caacctgccc 240

acccacggtc ctctggctgg tatgcgaatc cccatcatca acgagcacgg tgctgacgag 300

ctccgacgag agctcgagct gctcatgctc gcctccgaag aggacgagga agtctcctgt 360

ctgatcaccg atgctctgtg gtactttgcc cagtccgtcg ccgactctct caacctgcga 420

cgactcgttc tcatgacctc ctctctgttc aacttccacg cccacgtttc tctgccccag 480

tttgacgagc tcggttacct cgaccccgat gacaagaccc gactcgagga gcaggcttcc 540

ggtttcccca tgctcaaggt caaggacatc aagtccgcct actccaactg gcagattctc 600

aaggagattc tcggcaagat gatcaagcag accaaggcct cctccggtgt catctggaac 660

tccttcaagg agctcgagga gtccgagctc gagactgtca tccgagagat ccccgctccc 720

tctttcctca tccccctgcc caagcacctc accgcttcct cctcttctct gctcgaccac 780

gaccgaaccg tctttcagtg gctcgaccag cagccccctt cctccgtcct ctacgtttcc 840

ttcggctcca cctccgaggt cgacgagaag gacttcctcg agattgctcg aggcctcgtt 900

gactccaagc agtccttcct gtgggttgtc cgacccggct ttgtcaaggg ctccacctgg 960

gttgagcccc tgcccgatgg tttcctcggt gagcgaggcc gaattgtcaa gtgggtcccc 1020

cagcaggaag ttctggccca cggtgccatt ggtgccttct ggacccactc cggctggaac 1080

tccactctcg agtccgtctg cgagggtgtc cccatgatct tctccgactt tggcctcgac 1140

cagcccctca acgcccgata catgtccgat gttctcaagg tcggtgtcta cctcgagaac 1200

ggctgggagc gaggtgagat tgccaacgcc atccgacgag tcatggtcga cgaggaaggt 1260

gagtacatcc gacagaacgc ccgagtcctc aagcagaagg ccgatgtctc tctcatgaag 1320

ggtggttctt cttacgagtc tctcgagtct ctcgtttcct acatctcttc tttgtaa 1377

<210> 34
<211> 1419
<212> DNA
<213> Artificial sequence

<220>
<223> UGT2 variant optimized for expression in Y. lipolitica

<400> 34
atggctactt ccgactccat tgtcgacgac cgaaagaagc tccacattgt catgttcccc 60

tggctcgcct ttggccacat cattccctac ctcgagcttt ccaagctcat tgcccagaag 120

ggccacaagg tttctttcct ctccaccacc aagaacattg accgactctc ctcccacatc 180

tctcctctca tcaacgttgt ccagctcacc ctcccccgag tccaggagct gcccgaggac 240

gccgaggcca ccaccgatgt ccaccccgag gatatcccct acctcaagaa ggcctccgac 300

ggtctgcagc ccgaggtcac cgagttcctc gagcagcact ctcccgactg gatcatctac 360

gactacaccc actactggct cccctccatt gccaccaagc acggtgtctc tcgagcccac 420

ttctccgtca ccaccccctg ggccattgcc tacatgggcc ccactgctga cgccatgatc 480

aacggttccg atggccgaac cacccccgag gacttcactg tccctcccaa gtggttcccc 540

ttccccacca aggtctgctg gcgaaagcac gatctggccc gactcgttcc ctacaaggcc 600

cccggtatct ccgacggcta ccgaatgggt ctggtcatca agggctgcga ctgtctgctc 660

tccaagacct accacgagtt tggcacccag tggctccgac tcctcgagac tctccaccga 720

aagcccgtca tccccgtcgg tctgctccct ccctccatcc ccggctccga caaggacgac 780

tcttgggttt ccatcaagga gtggctcgac ggccaggaga agggctctgt tgtctacgtt 840

gctctcggtt ccgaggttct cgtcacccag gacgaggttg ttgagctggc ccacggtctg 900

gagctgtccg gcctcccctt cgtctgggct taccgaaacc ccaaggtccc cgccaagtcc 960

gactccgtcg agcttcccga tggtttcgtc gagcgagtcc gagatcgagg tctggtctgg 1020

acctcttggg ctccccagct ccgaatcctc tcccacgagt ccgtctgtgg tttcctcacc 1080

cactgcggtt ccggctccat cgtcgagggt ctgatgttcg gccaccccct catcatgctc 1140

cccatcttcg gtgaccagcc cctcaacgcc cgactccttg aggacaagca ggtcggtatc 1200

gagatccccc gaaacgaaga ggacggttcc ttcacccgag actctgttgc tgagtctctc 1260

cgactcgtca tggtcgagga agagggtaag atctaccgag agaaggccaa ggagatgtcc 1320

aagctgttcg gtgacaagga tctccaggac cagtacgtcg acgactttgt cgagtacctc 1380

cagaagcacc gacgagctgt tgccattgac cacgagtct 1419